# EUROPEAN PATENT SPECIFICATION

(11) **EP 2 359 139 B1**
(45) Date of publication and mention of the grant of the patent: **07.03.2018**
(21) Application number: 09825537.5
(22) Date of filing: 09.11.2009
(51) Int. Cl.: G01N 33/564, G01N 33/68, G01N 33/50

(54) **DIAGNOSIS AND TREATMENT OF AUTOMMUNE DISEASES BY TARGETING AUTOMMUNE-RELATED B CELLS ("ABCS")**
DIAGNOSE UND BEHANDLUNG VON AUTOIMMUNERKRANKUNGEN MITTELS ABZIELUNG AUF AUTOIMMUN-ASSOSZIIERTE B-ZELLEN (ABCS)
DIAGNOSTIC ET TRAITEMENT DES MALADIES AUTO-IMMUNES PAR CIBLAGE DES CELLULES B AUTO-IMMUNES (« CBAI »)

(30) Priority: 07.11.2008 US 112582 P
(43) Date of publication of application: 24.08.2011
(73) Proprietor: National Jewish Health, Denver, CO 80206 (US)
(72) Inventor: RUBTSOV, Anatoly, Denver,CO 80220 (US); KAPPLER, John, Denver,CO 80207 (US); MARRACK, Philippa, Denver,CO 80207 (US)
(74) Representative: Evens, Paul Jonathan
(86) International application number: PCT/US2009/063687
(87) International publication number: WO 2010/054288

(56) References cited:
- WO-A1-91/13974
- WO-A2-02/22212
- WO-A2-2006/121852
- WO-A2-2008/121876
- US-A1- 2004 191 756
- US-A1- 2004 191 756
- US-A1- 2004 202 658
- US-A1- 2004 202 658
- US-A1- 2006 040 288
- US-A1- 2006 040 288
- S. MIYAWAKI ET AL.: 'Splenic Lymphoma with villous lymphocytes with CD5+, CDllc + B-cell phenotype.' INTERNAL MEDICINE. vol. 32, no. 6, June 1993, pages 472 - 475, XP008151273
- C. SCHEINECKER ET AL.: 'Alterations of dendritic cells in systemic lupus erythematosus: phenotypic and functional deficiencies.' ARTHRITIS RHEUM. vol. 44, no. 4, April 2001, pages 856 - 865, XP008150293
- N. TEIG ET AL.: 'Age-related changes in human blood dendritic cell subpopulations.' SCAND. J. IMMUNOL. vol. 55, no. 5, May 2002, pages 453 - 457, XP008150148

## Description

### CROSS-REFERENCE TO RELATED APPLICATIONS

This application claims the benefit of priority from U.S. Provisional Application Serial No. 61/112,582, filed November 7,2008.

### FIELD OF THE INVENTION

The field of the present invention is diagnosis and compositions for use in treatment of autoimmune diseases based on the identification of a novel sub-population of B cells called Autoimmune- or Age-related B cells ("ABCs"), which express the CD11c cell surface protein.

### BACKGROUND OF THE INTENTION

Human beings and other vertebrates get autoimmune diseases such as rheumatoid arthritis, lupus and juvenile diabetes. These diseases occur because the immune system of the host, which is designed to attack and destroy infections, instead turns on the tissues of its own host and destroys them. In 2001, NIH estimated that about 5% of the population in the USA suffers from some type of autoimmune disease with a cost to the taxpayer of about $100 billion/year.

There are more than 80 different so-called autoimmune diseases in human beings, each defined by, amongst other things, the tissue being attacked. In juvenile diabetes, for example, the immune system destroys the beta cells of the pancreas, the cells that are responsible for production of insulin. In multiple sclerosis the immune system attacks cells in the brain, and in rheumatoid arthritis, the immune response causes inflammation and destruction of the joints. In patients with lupus the immune system makes antibodies against DNA and other material in the nuclei of all cells. These antibodies bind their targets and cause problems in various organs in the body, for example, the kidneys, because the combination of the antibodies and their targets causes, amongst other things, inflammation, which leads to tissue damage and malfunction.

To a large extent predisposition to autoimmune disease is genetically inherited. Amongst identical twins, for example, if one twin is diagnosed with an autoimmune disease, there is a 14-60% likelihood that the other member of the pair will also get the disease (Jarvinen and Aho, 1994). Also, gender plays a role in the development of disease. In lupus, females are 10 times more likely to get the disease than males (Zandman-Goddard et al., 2007), whereas for ankylosing spondylitis, a disease that attacks the spine, males are 3 times more likely to be sufferers than females.

In spite of the fact that lupus and other autoimmune diseases are in part genetically inherited, it is still difficult to predict which individuals are likely to get the disease and which are not. Disease in close family relatives is an indicator, but by no means infallible. For example, although close family relatives of a patient with lupus are 25 times more likely to get the disease than the general population, still only about 2% of close family relatives of a patient actually develop the disease. It would be valuable to have means of predicting whether or not an individual is going to develop an autoimmune disease, so that physicians intervention is possible before the disease appears, thus either preventing the disease altogether, or reducing its damaging effects.

There are currently few assays which predict which individuals will become autoimmune before the pathology of the disease occurs. In juvenile diabetes, a test for antibodies to insulin and other material can predict disease to some extent before the individual becomes diabetic. However, for lupus and rheumatoid arthritis, although affected individuals develop autoantibodies, these often do not appear until after the disease process has begun. Thus, there is a need in the art for simple and reliable methods to predict the onset of autoimmune diseases.

WO 91/13974 discloses methods and compositions for the treatment of B cell disorders through the use of antibody that selectively binds to the surface immunoglobulin of a target B cell, in combination with an immunoconjugate comprising at least one therapeutic agent coupled to an antibody that selectively binds to the CD19 antigen on B cells.

WO 2006/121852 discloses the treatment of autoimmune diseases and disorders using therapeutic antibodies that bind CD19 and mediate antibody-dependent-cell-mediated-cytotoxicity (ADCC).

WO 02/22212 discloses combination therapy for treating autoimmune diseases with the combination of an immunoregulator antibody (anti-B7.1, anti-B7.2 or anti-CD40L antibody) and at least one B cell antibody such as CD19, CD20, CD22, CD23 or CD37.

WO 2008/121876 discloses non-fucosylated therapeutic antibodies such as anti-CD20, anti-CD23 and anti-CD80 antibodies.

US 2004/0202658 discloses antagonists which bind to B cell surface markers, such as CD20, to treat autoimmune disease in mammals who experience an inadequate response to a TNF-alpha inhibitor.

US 2004/0040288 relates to compositions and methods for diagnosing, monitoring and/or treating an autoimmune or chronic inflammatory disease based on detecting the methylation status of markers, specifically, CD70 and CD40L.

US 2004/0191756 discloses homozygous knock-out mice lacking the Aiolos gene that exhibit multiple phenotypes in common with humans suffering form Systemic Lupus Erythematosus (SLE) and methods of screening for agents active against SLE.

### SUMMARY OF THE INVENTION

The scope of the invention is defined by the appended claims. In accordance with one aspect of the present invention, there is provided a composition for use in treating an autoimmune disease in a subject by reducing the activity of autoimmune-associated B cells (ABCs) present in the subject, wherein the ABCs are characterized by at least the presence of marker CD11c, wherein the composition comprises an agent that is an antibody or antibody fragment that specifically binds to a protein expressed by the ABCs and wherein the protein is CD11c, and at least one antibody or antibody fragment that specifically binds an additional protein selected from the group consisting of CD11b, B220, CD19, a surface Ig, CD80, CD86, MHC class II, CD5, CCL3, CXCL10, CCL19, CXCL9, granzyme A, and perforin.

In accordance with another aspect of the present invention, there is provided method of diagnosing an autoimmune disease in a subject, comprising:detecting the presence of autoimmune-associated B cells ("ABCs") in a test sample, wherein the ABCs comprise B cells that express the protein CD11c and one or more additional marker proteins, wherein detecting the presence of ABCs in the sample comprises detecting the cells that express CD11c and one or more additional marker proteins by detecting the expression of CD11c and the one or more additional marker proteins in the test sample, wherein expression of CD11c and the one or more additional marker proteins indicates the presence of ABCs in the test sample; and wherein the presence of ABCs in the sample at an elevated level as compared to a baseline level established from a control sample, identifies the subject as having or likely to develop the autoimmune disease.

In the method, the one or more additional marker proteins may be selected from the group consisting of: CD11b, B220, CD 19 and a cell surface Immunoglobulin Ig, wherein the surface Ig is selected from the group consisting of: IgG, IgM, IgA and IgE.

In the method, the one or more additional marker proteins may be selected from the group consisting of: CD80, CD86, MHC class II, CD5, CCL3, CXCL10, CCL19, CXCL9, granzyme A and perforin.

In the method, the ABCs may express low levels of CD21 as compared to other B cells.

In the method, detecting the expression of CD11c and the one or more additional marker proteins may comprise co-immunostaining the cells with an antibody or antibody fragment that specifically recognizes CD11c, and an antibody or antibody fragment that specifically recognizes the additional marker protein.

In the method, detecting the expression of CD11c and the one or more additional marker proteins may comprise detecting the mRNA levels of CD11c and the additional marker protein.

The method may further comprise determining the frequency of the cells that express the protein CD11c and the additional marker protein.

In the method, the test sample may be a blood sample comprising peripheral blood cells.

In accordance with yet another aspect of the present invention, there is provided use of a composition comprising an agent that is an antibody or antibody fragment that specifically binds to a protein expressed by autoimmune-associated B cells (ABCs) wherein the protein is CD11c and wherein the composition comprises at least one antibody or antibody fragment that specifically binds an additional protein selected from the group consisting of CD11b, B220, CD19, a surface Ig, CD80, CD86, MHC class II, CD5, CCL3, CXCL10, CCL19, CXCL9, granzyme A, and perforin.

In accordance with still another aspect of the present invention, there is provided a method to evaluate the efficacy of a treatment of an autoimmune disease in a subject, comprising:a) detecting the presence of autoimmune-associated B cells (ABCs) characterized by at least the presence of marker CD11c in a test sample taken from the subject before administering the treatment; b) detecting the presence of ABCs in a test sample taken from the subject after administering the treatment; wherein detecting the presence of ABCs in the sample before and after administering treatment comprises detecting B cells that express CD11c and one or more additional marker proteins by detecting the expression of CD11c and the one or more additional marker proteins in the test sample, wherein expression of CD11c and the one or more additional marker proteins indicates the presence of ABCs in the test sample; and c) comparing the level of ABCs in the test sample taken from the subject before administering the treatment to the level of ABCs in the test sample taken from the subject after administering the treatment; wherein ABCs comprise B cells that express CD11c.

In any of the methods, the autoimmune disease may be selected from the group consisting of: lupus, rheumatoid arthritis, multiple sclerosis, insulin dependent diabetes mellitis, myasthenia gravis, Grave's disease, autoimmune hemolytic anemia, autoimmune thrombocytopenia purpura, Goodpasture's syndrome, pemphigus vulgaris, acute rheumatic fever, post-streptococcal glomerulonephritis, and polyarteritis nodosa.

### BRIEF DESCRIPTION OF THE DRAWINGS

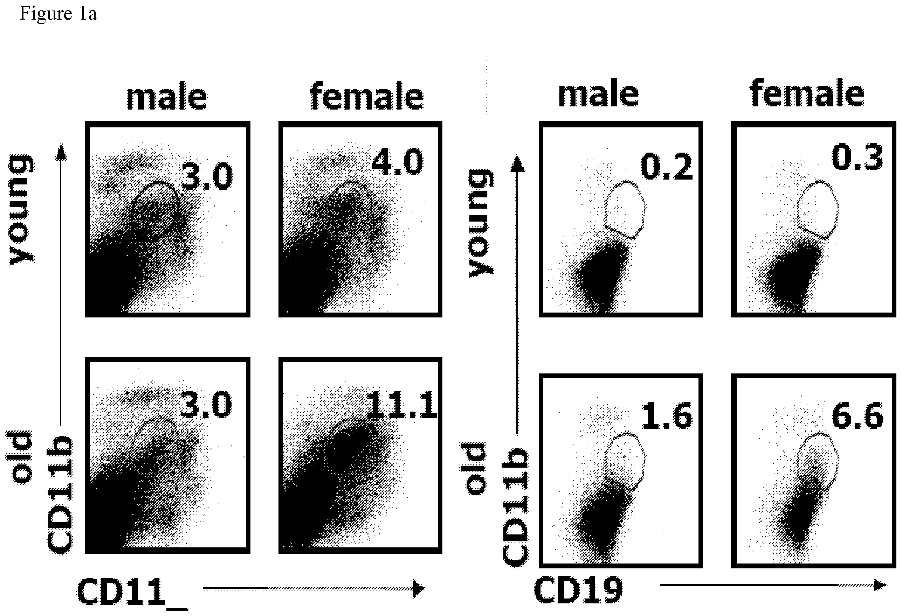

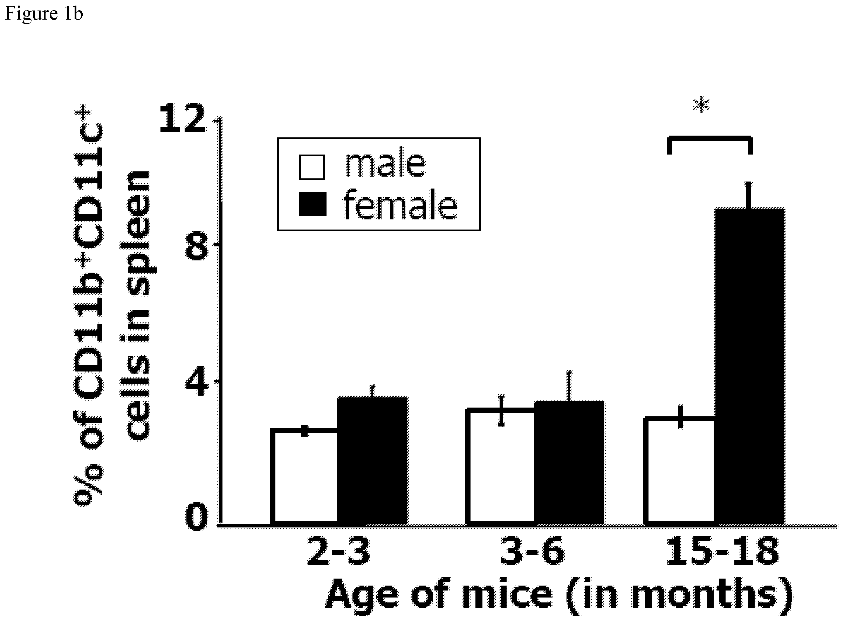

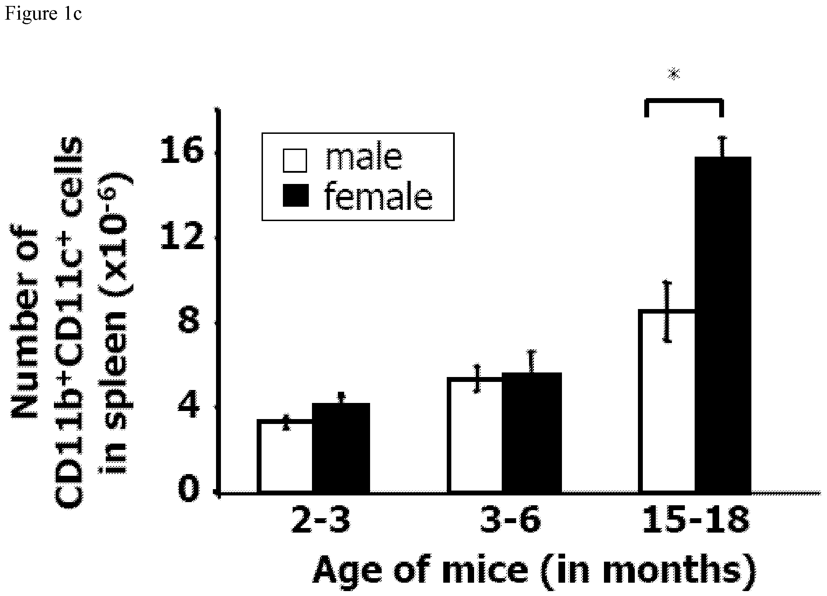

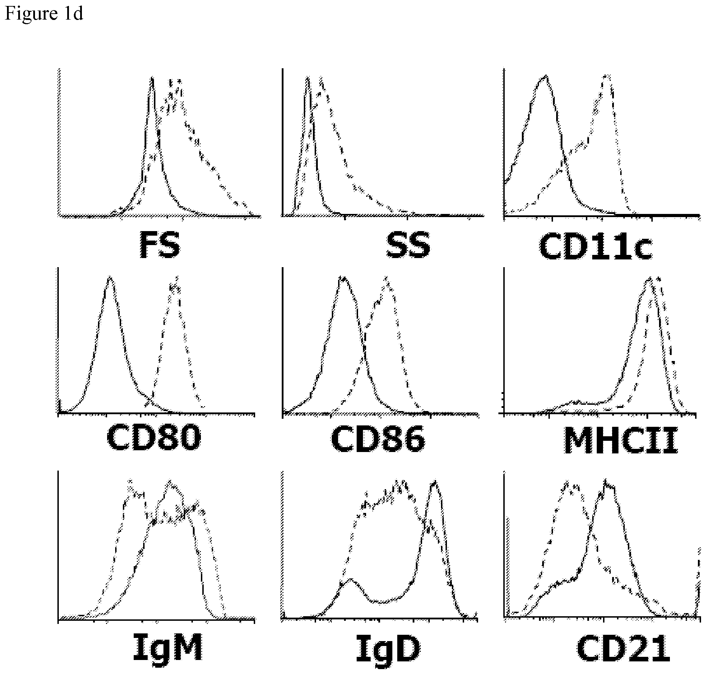

Figure 1 shows that Elderly female mice contain an enlarged population of CD19⁺CD11b⁺CD11c⁺ B cells (ABCs). Figure 1a shows the flow cytometric analysis of spleen or splenic B cells (gated as IgM⁺B220⁺CD4⁻CD8⁻NK1.1⁻) in young (<12 weeks old) and elderly (>1 year old) C57BL/6 mice. Data are representative of more than 5 independent analyses. Figure 1b and 1c show the average percent and number of CD19⁺CD11b⁺CD11c⁺ cells, respectively in the spleen of male (dark bars) and female (light bars) C57BL/6 mice. *, *P<0.01* (Students two tailed t-test). Figure 1d shows the flow cytometry of FO B cells (CD19⁺CD11b⁻) (black) and CD19⁺CD11^{b}+CD11c⁺ B cells (grey).

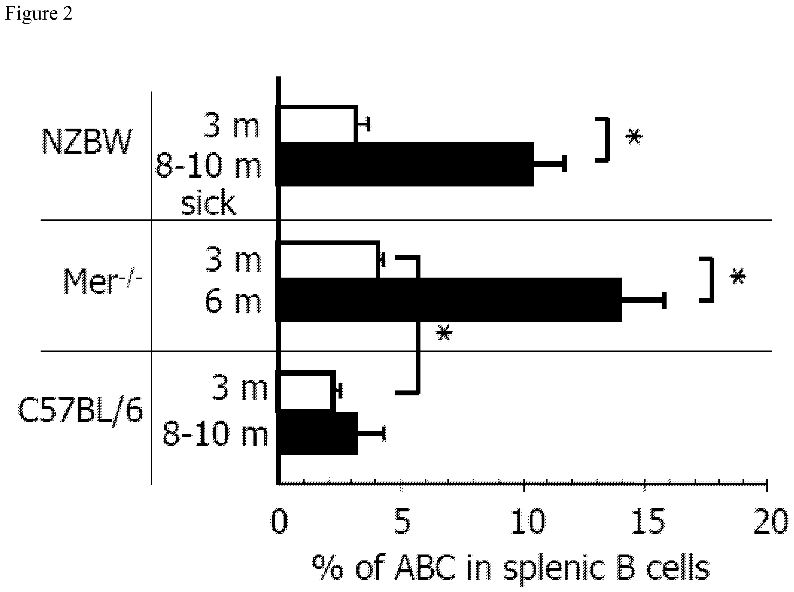

Figure 2 shows the increase in the number of ABCs in autoimmune prone mice at the time of onset of autoimmunity. Bars represent mean (± SEM) of at least 5 mice per group. *, *P<0.01* (Students two tailed t-test).

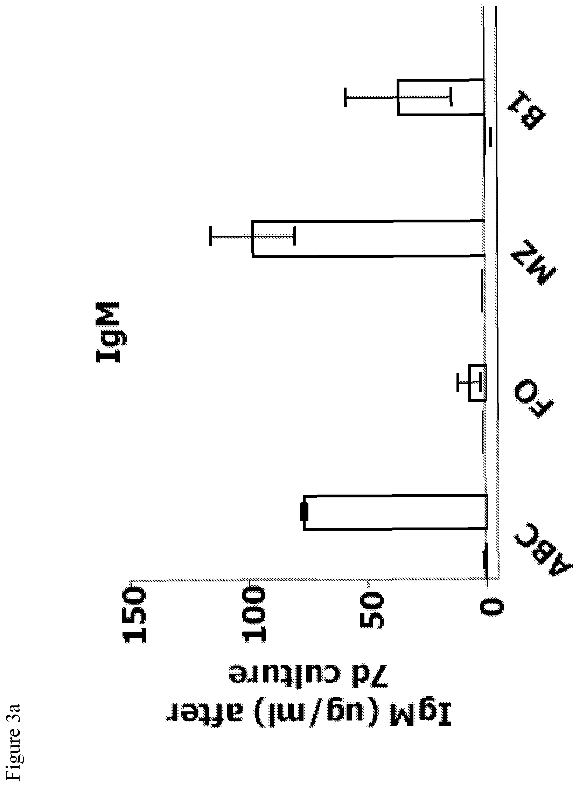

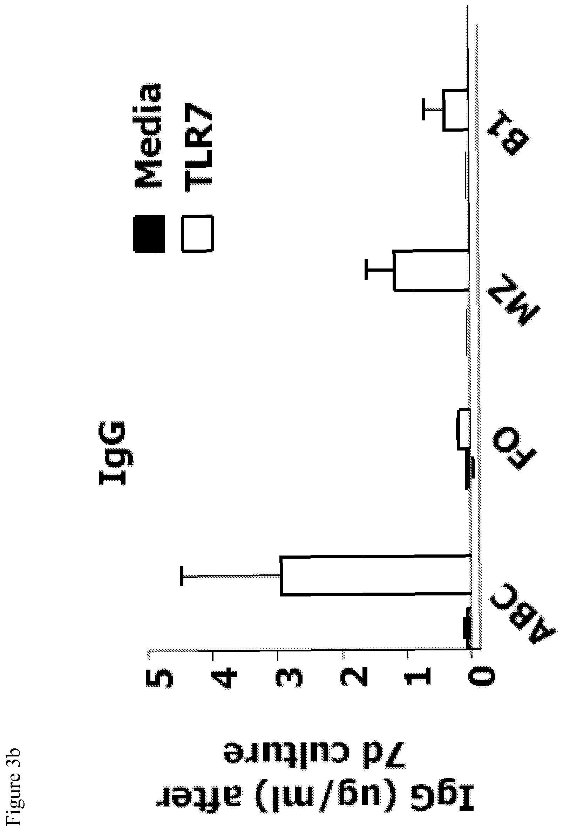

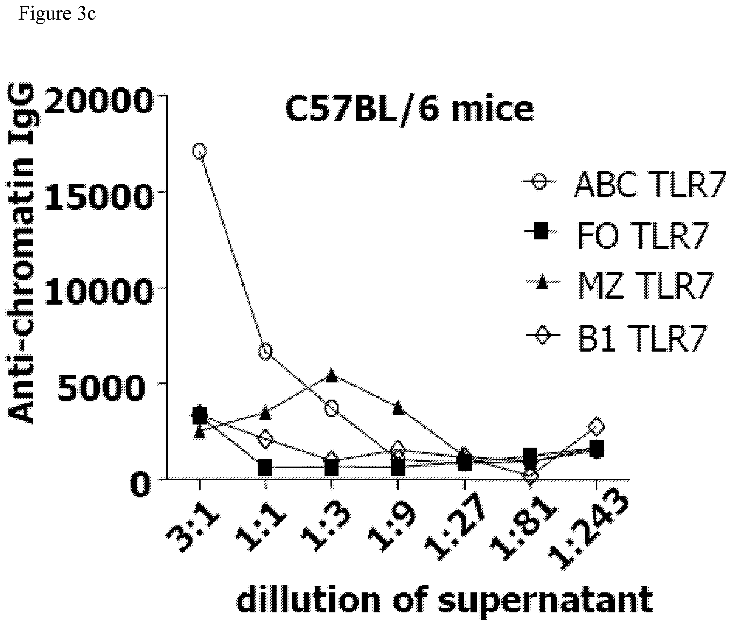

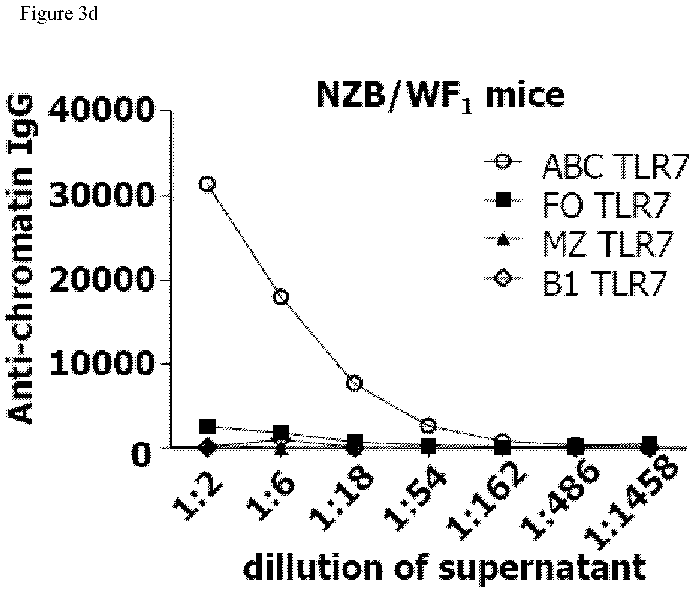

Figure 3 shows that ABCs produce anti-chromatin antibodies upon stimulation in vitro. Figures 3a, and 3b show the total IgM and IgG, respectively, from ABCs, FO, MZ and B1 B cells isolated from C57BL/6 mice cultured in the presence of medium or TLR7 agonist. Bars represent mean (± SEM) of three independent experiments. Figures 3c and 3d show anti-chromatin IgG in ABCs, FO, MZ and B1 B cells cultured in the presence of TLR7 agonist and isolated from C57BL/6 mice or autoimmune prone NZB/WF1 mice, respectively. Data are representative of three independent experiments.

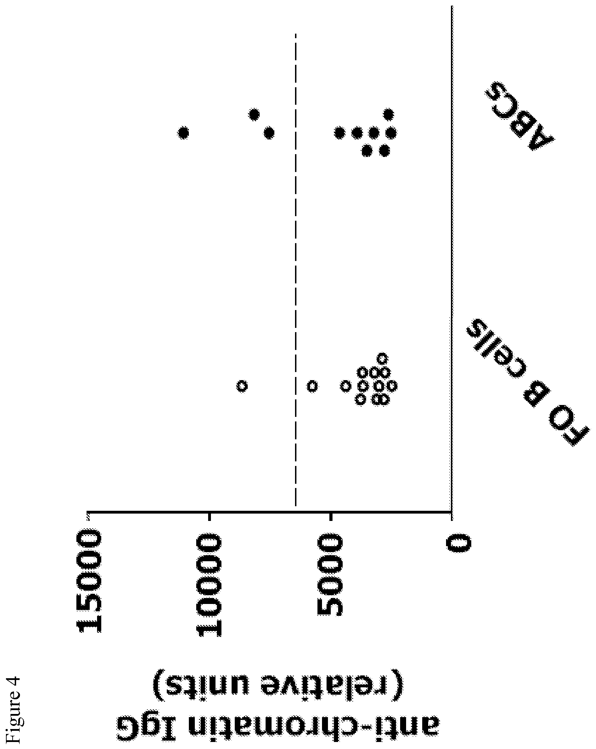

Figure 4 shows the frequency of anti-chromatin IgG in the supernatants of hybridomas (ABCs or FO B cells from aged C57BL/6 female mice fused with SP2/0 myeloma cells) as tested by ELISA for IgG production and chromatin reactivity. Dashed line indicates 2x the average reading obtained from assays of wells containing no primary antibody.

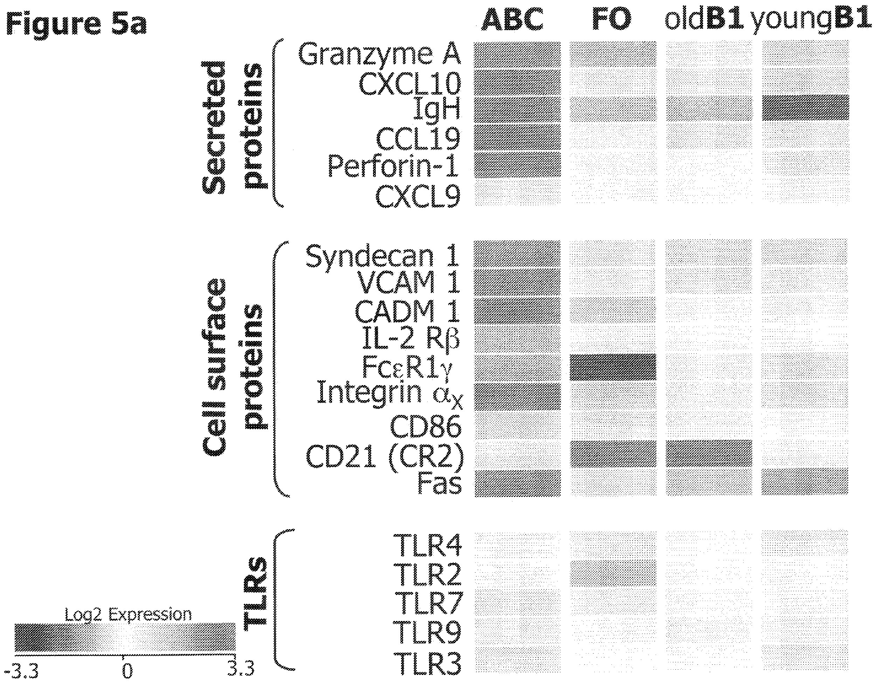

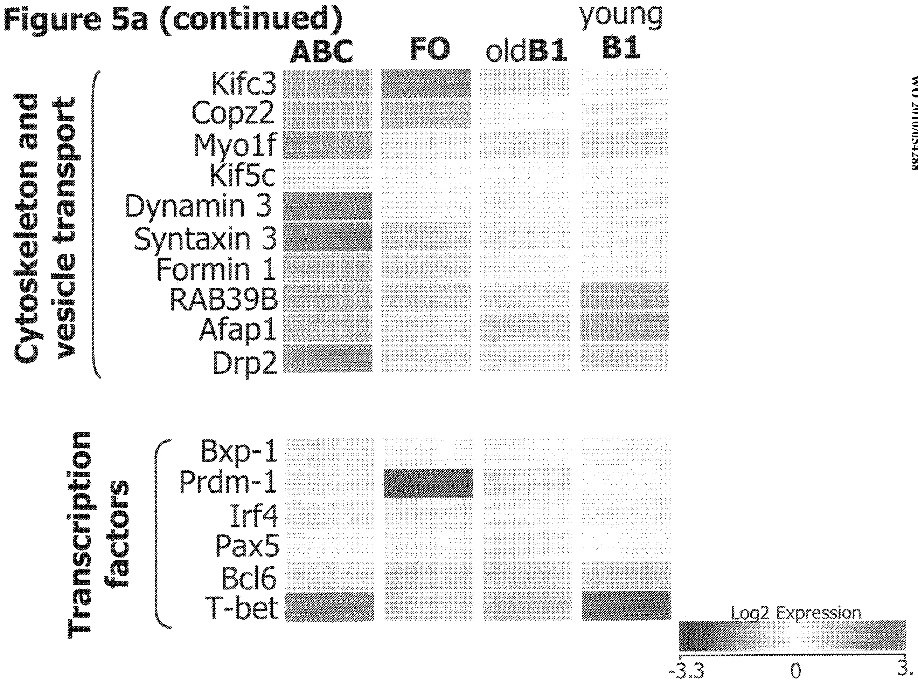

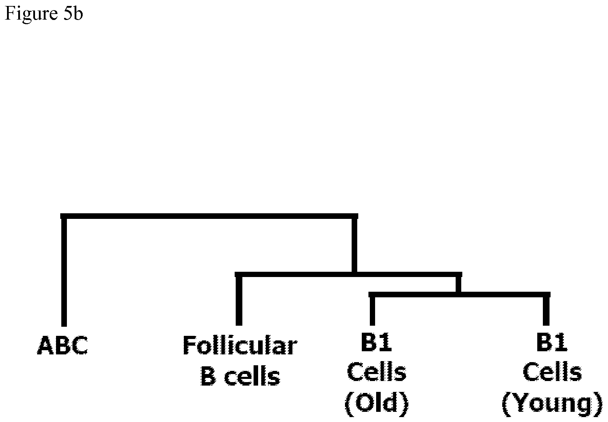

Figure 5 shows the Transcriptome analysis of ABCs, FO, MZ, and B1 B cells. In Figure 5a a selected list of genes upregulated by ABCs alone with some control genes is displayed. Up- and down- regulated transcripts, as well as the magnitude of expression is depicted by the Log2 Expression bar. Figure 5b shows the genealogical tree created by GeneSpring software based on gene expression profile of analyzed B cell populations.

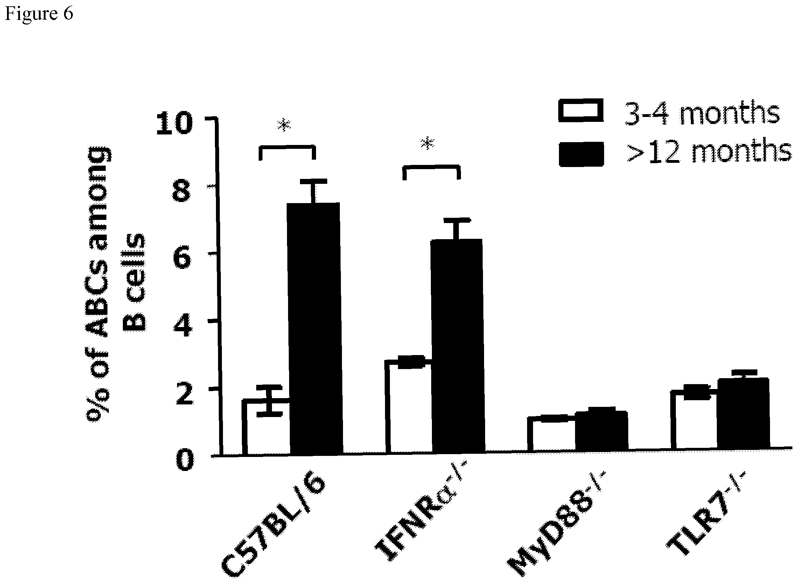

Figure 6 shows the average percentage of ABCs among B cell in spleen of young (12-16 weeks old) and aged (>12 months old) C57BL/6, IFNR^{-/-}, TLR7^{-/-}, MyD88^{-/-} female mice illustrating that TLR7 and MyD88 signaling is required for ABCs accumulation. Bars represent mean (± SEM) of at least 10 mice per group. *, *P<0.01* (Students two tailed t-test).

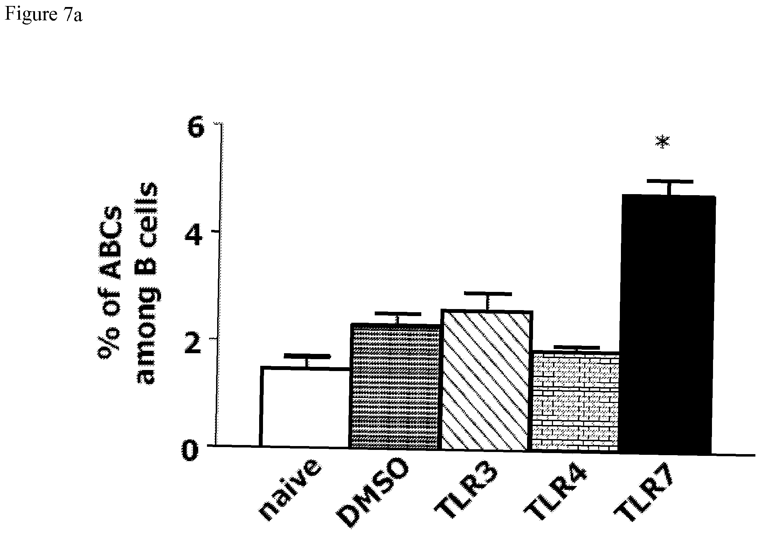

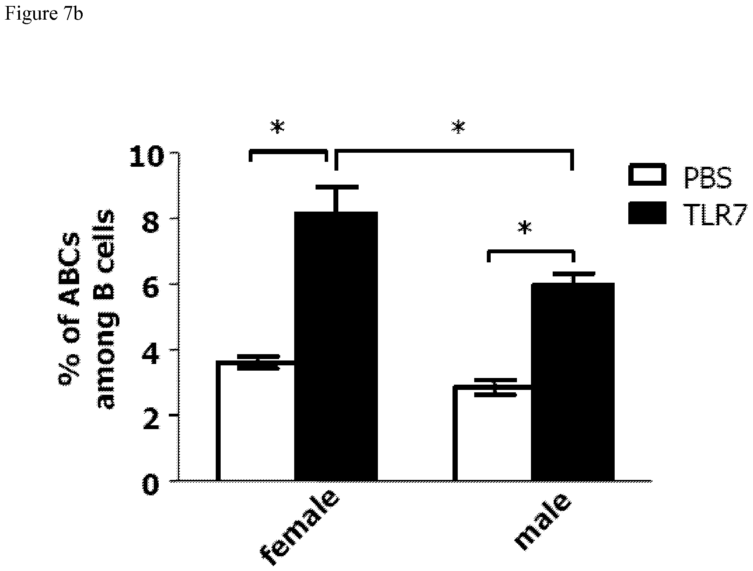

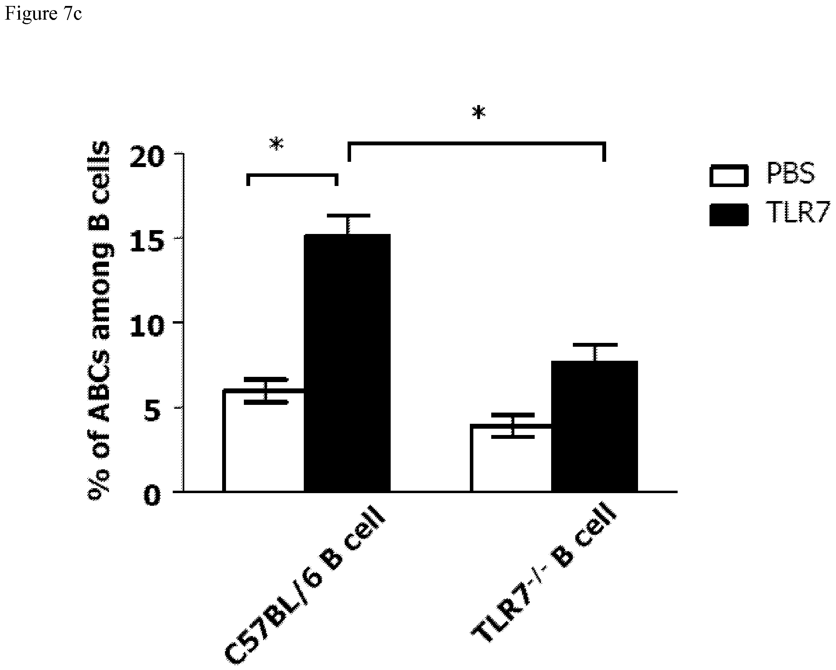

Figure 7 shows that chronic TLR7 stimulation is sufficient to induce ABCs accumulation. Figure 7a shows the average percentage of ABCs among B cells in spleen of young (8-12 weeks old) C57BL/6 female mice after 30 immunizations with vehicle or indicated TLR agonist. Figure 7b shows the percentage of ABCs in the spleen of young (8-12 weeks old) female and male C57BL/6 mice treated with vehicle or TLR7 agonist for 2 months. Bars represent mean (± SEM) of at least 5 mice per group. *, *P<0.05* (Students two tailed t-test).

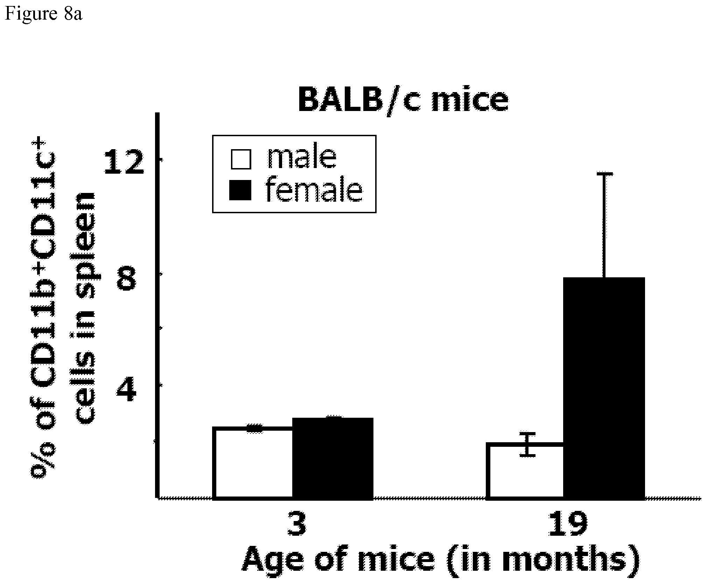

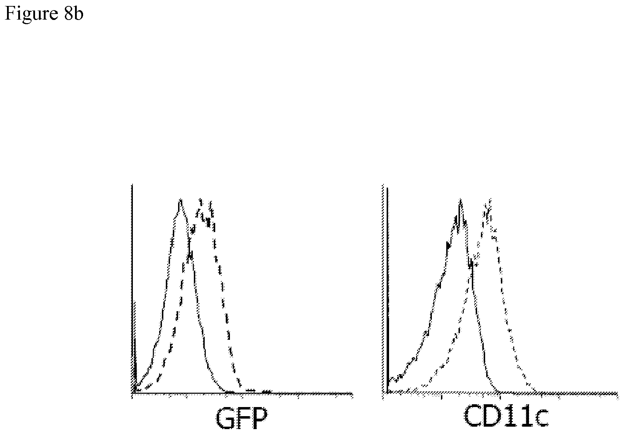

Figure 8a shows the average percent of CD19⁺CD11⁺CD11c⁺ cells in spleen of male and female BALB/c mice as determined by flow cytometric analysis. Figure 8b shows the detection of GFP (left) and anti-CD11c staining (right) in ABCs (gated as CD4-CD8⁻NK1.1⁻B220⁺CD19⁺CD11b⁺) from CD11c-DTR/GFP mice.

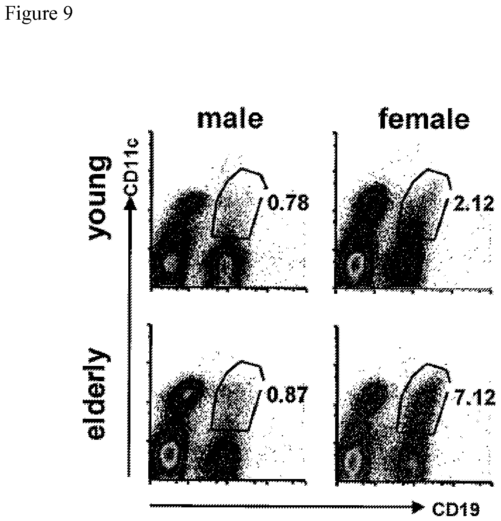

Figure 9 shows that elderly human females contain an expanded population of ABCs. the flow cytometric analysis of peripheral blood leukocytes in young (<30 weeks old) and elderly (>60 year old) healthy human volunteers to identify CD4⁻CD8⁻CD19⁺CD11c⁺B cells.

### DETAILED DESCRIPTION OF THE INVENTION

The present invention is directed to methods of diagnosis and compositions for use in treatment of autoimmune diseases. The invention is based on the discovery of a novel population of cells that appear in the blood and lymphoid organs of auto-immune prone mice. This population is made up of B cells, that express an unexpected collection of proteins on their surface including, most notably, a protein called CD11c. B cells are not normally thought to bear CD11c. These B cells are referred herein as Autoimmune-related or age-related B Cells or "ABCs." This population of ABCs also appears at high frequency in spleens of aged female wild type mice and may be part of the reason why females are more likely to become autoimmune than males. This population was also found to be present in elderly human females.

Additionally, the expression of the following proteins was observed in the ABCs: CD11b, B220, CD19, a cell surface Immunoglobulin Ig such as IgG, IgM, IgA and IgE, CD80, CD86, MHC class II, CD5, CCL3, CXCL10, CCL19, CXCL9, granzyme A and perforin. Additionally, ABCs were found to express low to undetectable levels of CD21 as compared to other B cells. The gene expression profile of the ABCs is described in detail in Example 4 and Figure 5.

A few populations of B cells bearing CD11c have been described by others. For example, some human B cell cancers, including hairy cell leukemias (Morice et al., 2008) and splenic marginal zone (MZ) B cell lymphomas (Kost et al., 2008) express CD11c, as do a subset of nontransformed human memory B cells, which might be involved in protection at mucosa (Ehrhardt et al,, 2008). A recent paper has shown that CD11c plasmablasts appear in mice in response to *Ehrlichia muris* infection (Racine et al., 2008). The CD11c⁺ ABCs described herein are not identical to any of these populations. Specifically, ABCs are not transformed, do not express a memory phenotype, are mainly found in the spleen and appear spontaneously with age rather than in response to infection.

ABCs are also not identical to two other unusual B cell populations that have recently been described. The high expression of CD80 and CD86 costimulation molecules, normal levels of B220 and expression of CD11c on ABCs distinguishes them from a previously characterized unusual B cell population in old mice (Johnson et al., 2002). In some respects ABCs share similarities with a novel population of activated memory, IgG expressing, B cells that appear in SLE patients (Nicholas et al., 2008). Although ABCs and this novel population both express elevated levels of CD19, low levels of CD21, and both are enriched in autoreactivity, the human memory cells have class switched immunoglobulin while ABCs do not possess memory phenotype and express IgM and IgD. ABCs also expressed some unexpected genes, such as granzyme A and perforin, suggesting that they may possess lytic function.

Interestingly, ABCs showed high expression of a number of genes whose protein products are involved in the cytoskeleton and/or in vesicle transport suggesting active secretion by this population, a property that might be relevant to the finding that ABCs contain mRNAs for several chemokines at much higher levels than found in follicular B cells and B1 cells. The arrays did not reveal over expression of any cytokines by ABCs in comparison with the other B cell populations. However, the cells studied in the array experiments were unstimulated. It is plausible that cytokine production by these cells, if it occurs, requires signaling through the BCR, while chemokine production, at least at the mRNA level, does not.

Furthermore, as described herein, ABCs are capable of secreting anti-chromatin IgG antibodies (Example 3). The ability of the ABCs to secrete autoreactive anti-chromatin antibodies indicates that they may be directly involved in the progression of autoimmunity. The high expression of MHC class II and costimulatory molecules on these cells suggests that ABCs may also present self antigens to T cells and may thus serve to initiate or enhance autoreactivity.

The elevation in the presence of ABCs is mediated by Toll-like receptor 7 ("TLR-7") and Myeloid differentiation primary response gene ("MyD88") signaling. The fact that virtually all female mice develop increased numbers of ABCs by 15 months of age suggests that their existence depends directly or indirectly on some property peculiar to females. Previous work by others had suggested that TLR7 may be a gender distinguishing factor since female mice have higher IFNα production than males in response to TLR7 stimulation (Berghofer et al., 2006). It is shown herein that ligands for TLR7, but not other TLRs, accelerate the appearance of ABCs (Example 5). As described in Example 5, young and old female mice deficient in the receptor for IFNαβ, IFNαR, or deficient in TLR7 were screened for the presence of ABCs in their spleens. TLR7^{-/-} aged female mice failed to accumulate ABCs. Likewise, ABCs did not accumulate in MyD88^{-/-}mice, which lack the key adaptor to initiate TLR7 signaling (Figure 6). These findings suggest that the accumulation of ABCs requires signaling through TLR7 and MyD88.

TLR7 is usually thought to bind viral single stranded RNA (Diebold, 2008), so it is probably frequently engaged in human beings but such viral products should not be present in our pathogen free mice. However, TLR7 has also been shown to bind host RNA (Diebold et al., 2006) suggesting that the stimulating ligand in old wild type mice comes from the animals themselves. This was supported by the finding reported herein that Mer-deficient mice, which have impaired apoptotic cell clearance, posses high number of ABCs at early ages and in both sexes. TLR7 activation leads to production of high amounts of IFNαβ (Hornung et al., 2005), cytokines that are thought to be important contributors to autoimmune diseases such as lupus (Alarcon-Segovia et al., 1974; Hooks et al., 1979; Jorgensen et al., 2007). However, normal development of ABCs in IFNαR deficient mice suggested that these cytokines may not be required for the generation of ABCs. Besides the induction of type I IFN production, TLR7 signaling was also shown to lead to production of IL-1, IL-6, IL-12 and tumor necrosis factor (TNF)-α; perhaps one or more of these proteins may be a crucial factor in the expansion of ABCs (Larange et al., 2009; Miller et al., 1999).

Accordingly, in one embodiment of the present invention, the present invention includes a method of diagnosing an autoimmune disease in a subject. One skilled in the art will readily appreciate that the method can be used to detect any autoimmune disease. Examples of such diseases include, without limitation, lupus, rheumatoid arthritis, multiple sclerosis, insulin dependent diabetes mellitis, myasthenia gravis, Grave's disease, autoimmune hemolytic anemia, autoimmune thrombocytopenia purpura, Goodpasture's syndrome, pemphigus vulgaris, acute rheumatic fever, post-streptococcal glomerulonephritis, and polyarteritis nodosa.

The term subject refers to any animal subject, and particularly, any vertebrate mammals, including, but not limited to, primates, rodents, livestock and domestic pets. Preferred mammals for the methods of the present invention include humans.

The term sample refers to any biological sample obtained from the subject that contains peripheral blood cells. The sample may be a biological fluid sample, such as blood. The sample may also be a tissue sample obtained from a lymph node or spleen biopsy.

The method includes the step of obtaining a test sample from a subject and detecting the presence of ABCs in the test sample, wherein the ABCs comprise B cells that express the protein CD11c. The presence of ABCs can be detected by identifying cells that express CD11c and one or more additional marker proteins. The additional marker protein may be any B cell marker protein or a protein that is expressed by the ABCs. The examples of additional marker proteins include, without limitation, CD11b, B220, CD19, a surface immunoglobulin (Ig) protein, CD80, CD86, MHC class II, CD5, CCL3, CXCL10, CCL19, CXCL9, granzyme A and perforin. In preferred embodiments, the additional markers that are used for detection may be CD11b, B220, CD19 and a surface Ig.

Expression of CD11c and one or more additional marker proteins may be assessed using any known methods in the art. The term expression refers to protein translation or mRNA transcription. Methods suitable for the detection of protein include any suitable method for detecting and/or measuring proteins from a cell or cell extract. Such methods include, but are not limited to, immunoblot (e.g., Western blot), enzyme-linked immunosorbant assay (ELISA), radioimmunoassay (RIA), immunoprecipitation, immunohistochemistry and immunofluorescence. Particularly preferred methods for detection of proteins include any single-cell assay, including immunohistochemistry and immunofluorescence assays. Such methods are well known in the art. Furthermore, antibodies against CD11c and the additional marker proteins described herein are known in the art and are described in the public literature, and methods for production of antibodies that can be developed against these proteins are also well known in the art.

Methods suitable for detecting mRNA include any suitable method for detecting and/or measuring mRNA levels from a cell or cell extract. Such methods include, but are not limited to: polymerase chain reaction (PCR), reverse transcriptase PCR (RT-PCR), in situ hybridization, Northern blot, sequence analysis, gene microarray analysis (gene chip analysis) and detection of a reporter gene. Such methods for detection of transcription levels are well known in the art, and many of such methods are described in detail in the attached examples, in Sambrook et al., Molecular Cloning: A Laboratory Manual, Cold Spring Harbor Labs Press, 1989 and/or in Glick et al., Molecular Biotechnology: Principles and Applications of Recombinant DNA, ASM Press, 1998.

In a preferred embodiment, the presence of ABCs is determined by co-immunostaining or co-immunolabeling the cells in the sample with an antibody or antibody fragment that specifically recognizes CD11c, and an additional antibody or antibody fragment that specifically recognizes an additional marker protein. In various embodiments, the cells may be co-immunostained using an anti-CD11c antibody and multiple (more than one) additional antibodies or antibody fragments, each of which recognizes a different marker protein. For example, the presence of ABCs may be detected by co-immunostaining cells for the expression of CD11c, CD11b, B220, CD19 and a cell surface Ig. The presence of ABCs may also determined by detecting the levels of mRNA for CD11c and the additional marker proteins.

The method may include the step of determining the frequency of the cells (percentage or the total number) that express both the protein CD11c and one or more additional marker proteins. The frequency may be determined by any known method. Such methods may include directly counting the immuno-positive cells with a hematocytometer. In a preferred embodiment, the frequency of the cells is determined by flow cytometry. Flow cytometry is a technique for counting and examining microscopic particles, such as cells, by suspending them in a stream of fluid and passing them by an electronic detection apparatus for simultaneous multiparametric analysis of the physical and/or chemical characteristics of the particles. A number of flow cytometers are commercially available and their operation and use is well known to one skilled in the art.

The presence of ABCs in the sample at an elevated level as compared to a baseline level established from a control sample, identifies the subject as having or likely to develop the autoimmune disease. A "baseline level" is a normal level of ABCs against which the level of ABCs in the sample is compared. Based on the control or baseline level of ABCs, it is determined whether a sample has an increased or elevated, decreased, or substantially the same level of ABCs. The term "negative control" or "normal control" used in reference to a baseline level typically refers to a baseline level established in a sample from the subject or from a population of individuals which is believed to be normal (i.e., non-disease or non-disease prone). A baseline can also be indicative of a positive diagnosis of the disease; such a baseline level is referred to as a "positive control" baseline and refers to a level of ABCs established in a sample from the subject, another subject or a population of subjects, wherein the subject or subjects were believed to be diseased or disease prone.

The baseline level of ABCs may be established from control samples, and preferably control samples that were obtained from a population of matched individuals. The phrase "matched individuals" refers to a matching of the control individuals on the basis of one or more characteristics which are suitable for the disease to be evaluated. For example, control individuals can be matched with the subject to be evaluated on the basis of gender, age, race, or any relevant biological or sociological factor that may affect the baseline of the control individuals and the subject (e.g., preexisting conditions, consumption of particular substances, levels of other biological or physiological factors). To establish a control or baseline level of ABCS, samples from a number of matched individuals are obtained and evaluated for ABCs levels. The sample type is preferably of the same sample type as the sample type to be evaluated in the subject. The number of matched individuals from whom control samples must be obtained to establish a suitable control level (e.g., a population) can be determined by those of skill in the art, but should be statistically appropriate to establish a suitable baseline for comparison with the subject to be evaluated (i.e., the test subject). The values obtained from the control samples are statistically processed using any suitable method of statistical analysis to establish a suitable baseline level using methods standard in the art for establishing such values.

It will be appreciated by those of skill in the art that a baseline need not be established for each assay as the assay is performed but rather, a baseline can be established by referring to a form of stored information regarding a previously determined baseline level of ABCs for a given control sample. Such a form of stored information can include, for example, but is not limited to, a reference chart, listing or electronic file of population or individual data regarding "normal" (negative control) or disease positive ABCs level; or a medical chart for the subject recording data from previous evaluations; or any other source of data regarding baseline ABCs level that is useful.

After the level of ABCs is determined in the sample, it is compared to the established baseline level of ABCs. Preferably, the method of determining the level of ABCs in the test sample is the same or qualitatively and/or quantitatively equivalent to the method used to establish the baseline level, such that the levels of the test sample and the baseline can be directly compared. In comparing the test sample to the baseline control, it is determined whether the test sample has a measurable decrease or increase in the level of ABCs over the baseline level, or whether there is no statistically significant difference between the test and baseline levels.

After comparing the levels of ABCs, the final step of making a diagnosis can be performed. Generally, a statistically significant increase in the level of ABCs as compared to the established baseline (i.e., with at least a 95% confidence level, or p<0.05), establishes a positive diagnosis of the autoimmune disease. Once a positive diagnosis is made using the present method, the diagnosis can be substantiated, if desired, using any suitable alternate method of detection of the disease.

Included in the present invention are kits for diagnosing an autoimmune disease in a subject. Such kit includes a reagent for detecting CD11c in a test sample (e.g., a probe that hybridizes under stringent hybridization conditions to a nucleic acid molecule encoding the CD11c; RT-PCR primers for amplification of mRNA encoding the CD11c or a fragment thereof; and/or an antibody, antigen-binding fragment thereof or other antigen-binding peptide that selectively binds to the CD11c). Such kit may also include additional reagents for detecting additional marker proteins that are expressed by the ABCs (e.g., a probe that hybridizes under stringent hybridization conditions to a nucleic acid molecule encoding a protein marker; PCR primers which amplify such a nucleic acid molecule; and/or an antibody, antigen binding fragment thereof, or antigen binding peptide that selectively binds to the control marker in the sample). Examples of suitable additional marker proteins have been described in detail in this application.

The reagents of the kit of the present invention can be conjugated to a detectable tag or detectable label. Such a tag can be any suitable tag which allows for detection of the reagents of part (a) or (b) and includes, but is not limited to, any composition or label detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, texas red, rhodamine, green fluorescent protein, and the like), radiolabels (e.g., 3H, 125I, 35S, 14C, or 32P), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads.

In addition, the reagents of the kit can be immobilized on a substrate. Such a substrate can include any suitable substrate for immobilization of a detection reagent such as would be used in any of the previously described methods of detection. Briefly, a substrate suitable for immobilization of a means for detecting includes any solid support, such as any solid organic, biopolymer or inorganic support that can form a bond with the means for detecting without significantly effecting the activity and/or ability of the detection means to detect the desired target molecule. Exemplary organic solid supports include polymers such as polystyrene, nylon, phenol-formaldehyde resins, acrylic copolymers (e.g., polyacrylamide), stabilized intact whole cells, and stabilized crude whole cell/membrane homogenates. Exemplary biopolymer supports include cellulose, polydextrans (e.g., Sephadex®), agarose, collagen and chitin. Exemplary inorganic supports include glass beads (porous and nonporous), stainless steel, metal oxides (e.g., porous ceramics such as ZrO2, TiO2, Al2O3, and NiO) and sand.

There is also disclosed a method of treating an autoimmune disease in a subject comprising reducing the activity of the ABCs present in the subject. The activity of the ABCs may be reduced by selectively targeting and removing (killing or inactivating) these cells in the subject. This may be accomplished by any method known to one skilled in the art. For example, the subject may be administered with an antibody or antibody fragment that specifically binds to a protein expressed on the surface of the ABCs, such as, without limitation, CD11c. Such binding would lead to removal or inactivation of that ABC.

In another aspect, the step of reducing the activity of the ABCs may comprise suppressing or inhibiting the ability of the ABCs to secrete anti-chromatin IgGs. This may comprise inhibiting the TLR7 and MyD88 mediated signaling. Thus, in some embodiments, this step may include reducing the activity or expression of TLR7. Methods for doing so would be readily apparent to one skilled in the art. For example, reducing TLR7 activity or expression can be accomplished by administering to the ABCs a TLR7 inhibitor. The inhibitor may be a protein, nucleic acid molecule, antibody, or a compound that is a product of rational drug design (i.e., drugs) that decreases the activity or expression of TLR7.

The inhibitor may be a protein that binds to TLR7 and inhibits the TLR7-MyD88 signaling. For example, the inhibitor may be an antibody or an antibody fragment that selectively binds to TLR7. In some embodiments, the inhibitor may be a chemical compound or drug that is an antagonist of TLR7. Such antagonists are known in the art and many are commercially available. Examples of TLR7 antagonists include, without limitation, IMO-3100, chloroquine, hydroxychloroquine and quinacrine. TLR7 antagonists may include DNA-based compounds. For example, it has been reported that 2'OMe-modified RNA functions as an inhibitor of TLR7 (Robbins, Molecular Therapy 2007 Sep; 15(9):1663-9. Epub 2007 Jun 19).

Methods for reducing expression of a protein are also well known in the art. Reduction of TLR7 expression may be at the transcriptional, translational or post-translational level. In a preferred embodiment, this may comprise administering to the subject TLR7 antisense oligonucleotide that specifically inhibits the expression of TLR7.

The TLR7 inhibitor (a molecule that is capable of reducing the activity or expression of TLR7), may be administered with a pharmaceutically acceptable carrier, which includes pharmaceutically acceptable excipients and/or delivery vehicles, for delivering the inhibitor to a subject (e.g., a liposome delivery vehicle). As used herein, a pharmaceutically acceptable carrier refers to any substance suitable for delivering a therapeutic composition useful in the method of the present invention to a suitable in vivo or ex vivo site. Preferred pharmaceutically acceptable carriers are capable of maintaining the inhibitor in a form that, upon arrival of the inhibitor to a target cell, the inhibitor is capable of entering the cell and decreasing the TLR7 activity or expression in the cell. Suitable excipients of the present invention include excipients or formularies that transport or help transport, but do not specifically target a nucleic acid molecule to a cell (also referred to herein as non-targeting carriers). Examples of pharmaceutically acceptable excipients include, but are not limited to water, phosphate buffered saline, Ringer's solution, dextrose solution, serum-containing solutions, Hank's solution, other aqueous physiologically balanced solutions, oils, esters and glycols. Aqueous carriers can contain suitable auxiliary substances required to approximate the physiological conditions of the recipient, for example, by enhancing chemical stability and isotonicity. Suitable auxiliary substances include, for example, sodium acetate, sodium chloride, sodium lactate, potassium chloride, calcium chloride, and other substances used to produce phosphate buffer, Tris buffer, and bicarbonate buffer. Auxiliary substances can also include preservatives, such as thimerosal, m- or o-cresol, formalin and benzol alcohol. Compositions of the present invention can be sterilized by conventional methods and/or lyophilized.

One type of pharmaceutically acceptable carrier includes a controlled release formulation that is capable of slowly releasing a composition of the present invention into an animal. As used herein, a controlled release formulation comprises the inhibitor in a controlled release vehicle. Suitable controlled release vehicles include, but are not limited to, biocompatible polymers, other polymeric matrices, capsules, microcapsules, microparticles, bolus preparations, osmotic pumps, diffusion devices, liposomes, lipospheres, and transdermal delivery systems. Natural lipid-containing delivery vehicles include cells and cellular membranes. Artificial lipid-containing delivery vehicles include liposomes and micelles. A delivery vehicle of the present invention can be modified to target to a particular site in a subject, thereby targeting and making use of a nucleic acid molecule at that site. Suitable modifications include manipulating the chemical formula of the lipid portion of the delivery vehicle and/or introducing into the vehicle a targeting agent capable of specifically targeting a delivery vehicle to a preferred site, for example, a preferred cell type.

Preferred routes of administration will be apparent to those of skill in the art, depending on the type of delivery vehicle used, whether the compound is a protein, nucleic acid, or other compound (e.g., a drug) and the level of disease or condition experienced by the subject. Preferred methods of in vivo administration include, but are not limited to, intravenous administration, intraperitoneal administration, intramuscular administration, intracoronary administration, intraarterial administration (e.g., into a carotid artery), subcutaneous administration, transdermal delivery, intratracheal administration, subcutaneous administration, intraarticular administration, intraventricular administration, inhalation (e.g., aerosol), intracerebral, nasal, oral, pulmonary administration, impregnation of a catheter, and direct injection into a tissue. These administrations can be performed using methods standard in the art. Oral delivery can be performed by complexing a therapeutic composition of the present invention to a carrier capable of withstanding degradation by digestive enzymes in the gut of an animal. Examples of such carriers, include plastic capsules or tablets, such as those known in the art. One method of local administration is by direct injection. Administration of a composition locally within the area of a target cell refers to injecting the composition centimeters and preferably, millimeters from the target cell or tissue. The inhibitor may be provided in any suitable form, including without limitation, a tablet, a powder, an effervescent tablet, an effervescent powder, a capsule, a liquid, a suspension, a granule or a syrup.

An effective administration protocol (i.e., administering a composition in an effective manner) comprises suitable dose parameters and modes of administration that result in some measurable, observable or perceived benefit to the subject from such administration. Effective dose parameters can be determined by experimentation using in vitro cell cultures, in vivo animal models, and eventually, clinical trials if the subject is human. Effective dose parameters can be determined using methods standard in the art for a particular disease or condition that the subject has or is at risk of developing. Such methods include, for example, determination of survival rates, side effects (i.e., toxicity) and progression or regression of disease.

The present invention also includes a method to evaluate the efficacy of a treatment of an autoimmune disease in a subject. In this method, the levels of ABCs may be determined in a sample taken from the subject before and after administering the treatment, and the before and after levels compared. The level of ABCs after administering the treatment may be greater than before administering the treatment, less than before administering the treatment, or may remain about the same as before administering the treatment. Depending on the results of the comparison of the ABCs levels before and after administering the treatment, the treatment plan may be revised to provide better therapeutic outcome. The level of ABCs after administering the treatment may be monitored over a period of time. The monitoring may continue even after the initial treatment plan has ended to detect whether the disease has returned. Preferably, the method of detecting the level of ABCs before and after administering the treatment is the same.

In some embodiments, the baseline level can be established from a previous sample from the subject being tested, so that the disease of a subject can be monitored over time and/or so that the efficacy of a given therapeutic protocol can be evaluated over time. In such embodiments, the baseline level of ABCs is determined from at least one measurement of ABCs in a previous sample from the same subject. Such a sample is from the subject at a different time point than the sample to be tested. In one embodiment, the previous sample may have resulted in a negative diagnosis (i.e., no disease, or potential therefor, was identified). In this embodiment, a new sample is evaluated periodically (e.g., at annual physicals), and as long as the subject is determined to be negative for the disease, an average or other suitable statistically appropriate baseline of the previous samples can be used as a "negative control" for subsequent evaluations. For the first evaluation, an alternate control can be used, as described below, or additional testing may be performed to confirm an initial negative diagnosis, if desired, and the value for the level of ABCs can be used thereafter. This type of baseline control is frequently used in other clinical diagnosis procedures where a "normal" level may differ from subject to subject and/or where obtaining an autologous control sample at the time of diagnosis is not possible, not practical or not beneficial. In another embodiment, the previous sample from the subject may have resulted in a positive diagnosis (i.e., the disease was positively identified). In this embodiment, the baseline provided by the previous sample is effectively a positive control for the disease, and the subsequent samplings of the subject are compared to this baseline to monitor the progress of the disease and/or to evaluate the efficacy of a treatment that is being prescribed for the disease. In this embodiment, it may also be beneficial to have a negative baseline level of ABCs (i.e., a normal cell baseline control), so that a baseline for regression of the disease can be set. Monitoring of a subject's disease can be used by the clinician to modify the disease treatment for the subject based on whether an increase or decrease in ABCs is indicated.

The invention now being generally described will be more readily understood by reference to the examples on the following pages, which are included merely for the purposes of illustration of certain aspects of the embodiments of the present invention. The examples are not intended to limit the invention, as one of skill in the art would recognize from the above teachings and the following examples that other techniques and methods can satisfy the claims and can be employed without departing from the scope of the claimed invention.

### EXAMPLES

### Example 1:

This example illustrates that a novel CD11c+ B cell population (termed ABCs) accumulate in the spleens of aged females.

Flow cytometric comparisons of hematopoietic cells from wild type C57BL/6 mice of either sex (obtained from The Jackson Laboratory) were performed at various ages. Cells were stained under saturating conditions with antibodies to mouse CD4 (clone GK1.5), CD8 (clone 53-6.7), CD5 (clone 53-7.3), B220 (clone RA3-6B2), IgM (clone R33-24), CD11b (clone M1/70), CD11c (clone N418), CD19 (clone 1D3), CD1d (clone 1B1), CD21 (clone 7G6), TCRαβ (clone H57-597), TER-119 (clone TER-119), CD138 (clone 281-2), CD80 (clone 16-10A1), CD86 (clone GL1), MHC-II (clone M5/114.15.2), IgD (clone 1-3.5) purchased from Ebiosciences or BD Pharmingen, or generated in house. Cells were analyzed by flow cytometry on CyAn (Beckman-Coulter) instrument and data were analyzed using FlowJo software (Treestar).

Flow cytometric analysis showed that the spleens of elderly female C57BL/6 animals contained significantly more CD11b⁺ CD11c⁺ cells than the spleens of male mice of the same age, or young mice of either sex (Figure 1A). Closer examination revealed that these were B220⁺, IgM⁺, CD11b⁺, CD11c⁺ and CD19⁺ (Figure 1A, D), therefore they were an unexpected and previously undescribed population of B cells, distinguishable from other cells in the spleen. The total number and the frequency of these cells was always higher in elderly female mice than in elderly males, or in young mice of either sex (Figure 1B,C). Given that the cells appear at high frequency in aged mice, these were named by the present inventors as Aged-associated B Cells, or ABCs. A substantial population of ABCs was also observed in aged female but not male BALB/c mice (Figure 8a.).

Further flow cytometric characterization of these cells revealed that they have higher forward and side scatter than follicular (FO) B cells and express high levels of CD80, CD86 and MHC class II, but are negative for CD21 (Figure 1D). ABCs were also found in lymph nodes and blood in mice over 2 years old (data not shown) perhaps due to their dissemination from spleen. Many of the surface markers of the ABCs, such as CD11b and CD5 are also expressed by B1 cells, normally found in the peritoneal cavity but also present in spleen. However, ABCs differed from B1 cells in CD11c expression and relatively high levels of B220 (Figure 1 and data not shown).

To confirm that ABCs express CD11c, which is generally considered to be a dendritic cell specific marker, cells were analyzed from CD11c-DTR/GFP mice (Jung et al., 2002). As determined by GFP expression, ABCs from aged female CD11c-DTR/GFP mice indeed express CD11c, confirming the specificity of the antibody staining and the unexpected phenotype of these B cells (Figure 8b).

### Example 2:

This examples shows that ABCs appear early in autoimmune prone mice, but not n healthy mice.

The frequency of ABCs in autoimmune prone strains was assessed at various ages. The F₁ hybrids of New Zealand Black and New Zealand White mice (NZB/WF₁) spontaneously develop a lupus-like autoimmune disease with strong similarities to human systemic lupus erythematosus (Kono and Theofilopoulos, 2000). As in humans, the onset and severity of lupus in this mouse model is accelerated in females. Flow cytometric analysis of 3 and 8-10 month old female NZB/WF₁ mice was performed. It is important to note that while 3 month old mice appeared healthy, all 8-10 month mice used in this experiment exhibited signs of disease progression, determined by the high level of protein in urine (data not shown). As shown in Figure 2, the percentage of ABCs in the spleen was slightly increased in healthy 3 month old NZB/WF₁, compared to that in age matched C57BL/6 mice. Moreover, 8-10 month old NZB/WF₁ mice with ongoing disease had a significant increase in the percentage of ABCs in the spleen compared to age matched C57BL/6 mice (Figure 2).

To confirm that these results were not model-specific and could be found in other autoimmune models, Mer^{-/-} mice were tested for the presence of ABCs in the spleen. (Mer^{-/-} mince on a C57BL/6 genetic background were a gift from Dr. Douglas Graham, UCHSC, Denver). In mice lacking the tyrosine kinase, Mer (Mer^{-/-}), there is inefficient uptake of apoptotic cells and these mice develop anti-nuclear antibodies (Scott et al., 2001). Similar to NZB/WF₁ mice, the appearance of autoantibodies in these mice is accelerated in females and can be detected by 6 months of age (Cohen et al., 2002). Analysis of 3 and 6 month old Mer^{-/-} mice for the presence of ABCs in spleens revealed that 3 month old mice had a 2 fold higher frequency of ABCs than age matched C57BL/6 mice (Figure 2). This was seen even though, at this age, the mice contain no detectable autoantibodies. The percentage of ABCs in Mer^{-/-} mice was dramatically higher in 6 month-old animals and was significantly higher than the percentage of ABCs in 10 month old C57BL/6 females (Figure 2).

These results show that the increase in the number of ABCs in the spleen correlates with, or even precedes, the development of systemic autoimmune disease.

### Example 3:

This example illustrates that the ABCs secrete anti-chromatin IgG antibodies in vitro.

To examine whether ABCs influenced the development of autoimmunity directly, ABCs, FO, MZ and B1 B cells, were isolated from aged wild type C57BL/6 females and stimulated with the TLR7 agonist, 3M-012 (Wille-Reece et al., 2005), a stimulus that is sufficient for B cell activation and antibody production (Rubtsov et al., 2008).

For isolation of the distinct B cell populations, following procedures were employed. Splenic B cells were purified by negative enrichment using biotinylated TER-119, NK1.1 and anti-TCRαβ antibodies followed by anti-biotin microbeads (Miltenyi, Germany). ABCs were purified with a MoFlo sorter (Dako-Cytomation) as B220⁺CD19⁺CD11b⁺ to greater than 95% purity and were verified for CD11c expression. FO B cells were identified as B220⁺CD19⁺CD11b⁻CD21^{int}CD1d^{int}, and Marginal Zone (MZ) B cells were isolated as B220⁺CD19⁺CD11b⁻CD21^{high}CD1d^{high}. To obtain B1 B cells, peritoneal cavity was washed with PBS and B1 B cells were purified as CD5⁺B220^{low}CD19⁺CD11b^{low}.

Concentrations of anti-chromatin IgG antibodies were determined using the protocol of Guth et al (Guth et al., 2009). For in vitro antibody production, ABCs, MZ, FO and BIB cells were incubated at 10⁶ cells/ml in complete DMEM media with or without TLR7 agonist 3M-012 1µg/ml). Supernatants were harvested at day 7 and the concentration of total and anti-chromatin IgG was determined by ELISA.

After 7 days in culture in the presence or absence of TLR7 agonist, supernatants were analyzed for secreted IgM and IgG. The results indicated that while all B cells were capable of secreting IgM, ABCs produced a relatively high amount of IgG after this stimulation (Figure 3A,B). IgM production by ABCs was lower than that of B1 cells and equivalent to the MZ B cell response. None of the B cell populations secreted immunoglobulins when cultured in media alone, suggesting that ABCs are not fully differentiated plasma cells and have to be stimulated to produce antibodies *in vitro.*

The same supernatants were tested for reactivity against chromatin and, as shown in Figure 3C, IgG produced by ABCs but not by other B cell populations showed anti-chromatin reactivity. To test whether ABCs from autoimmune prone mice can also secrete autoantibodies, B cell populations were sorted from 10 month old NZB/WF₁ female mice with ongoing disease and proteinurea and stimulated with TLR7 agonist *in vitro.* Again, only ABCs were able to secrete anti-chromatin IgG, while other B cell populations secreted predominantly IgM with no reactivity to chromatin (Figure 3D). It is important to note that the supernatants from *in vitro* experiments were concentrated for detection of the anti-chromatin autoantibodies produced by ABCs from C57BL/6 mice (Figure 3C), while the level of the anti-chromatin IgG secreted by ABCs from NZB/WF1 mice was easily detected at a 15 fold dilution (Figure 3D).

To confirm that ABCs can make chromatin-reactive autoantibodies, B cell hybridomas were made from aged C57BL/6 mice. Hybridomas were generated from ABCs and FO B cells. First, cells were isolated from spleens of aged (>15 months) female C57BL/6 mice and incubated for 3 days *in vitro* in the presence of anti-CD40 antibodies (10 ug/ml) and IL-4 (50 ng/ml). After *in vitro* culture, activated B cells were fused to SP2/0 myeloma cells as described previously (Haskins et al., 1983). The plates were seeded with cells at 0.5 x 10⁶ cells/ml in complete SMEM medium + recombinant IL-6 (500 U/ml) + 10% FBS and incubated at 37°C in 10% CO₂ for 24 h before starting selection with HAT media supplement (Sigma). Individual hybridomas were assessed for production of total and anti-chromatin IgG by ELISA.

As shown in Figure 4, 3 out of 10 (30%) IgG-secreting hybridoma clones derived from ABCs secreted anti-chromatin IgG, while only 1 of the 13 (7.5%) FO-derived IgG-secreting clones showed chromatin reactivity.

Together, these results demonstrated that ABCs can be the source of autoantibodies and suggest that the increase in the size of the ABC population in autoimmune susceptible mice might directly influence the onset of autoimmunity.

### Example 4:

This examples illustrates the pattern of gene expression in ABCs.

To further characterize the new B cell population and to compare the properties of ABCs with those of other B cell populations in more detail, gene array analysis was performed.

Total RNA from at least 500,000 cells from each purified population was extracted using the PicoPure RNA Isolation Kit (Arcturus), and RNA integrity was assessed using a bioanalyzer (Agilent Technologies). Fragmented, labeled RNA samples were then hybridized overnight onto Affymetrix mouse genome 430 2.0 microarray, containing 45,101 probe sets. Analysis of microarray results was done by using GeneSpring X (Agilent Technologies). The normalized hybridization intensity for each probe set was calculated using the GC-RMA method implemented in the GeneSpring software package (Agilent Technologies) as the default setting. Genes whose expression was increased (threshold 2.0) within ABCs compared to the other B cell populations were subjected to one-way statistical test, using a Welch *t* test (parametric test, variances not assumed equal), with a *P* value cutoff of 0.05.

Gene expression in ABCs (sorted as B220⁺CD19⁺CD11b⁺ and verified for CD11c expression) was compared with FO B cells (sorted as B220⁺ CD19⁺CD11b⁻ CD1d^{int} CD21^{int}) purified from the spleens of elderly female C57BL/6 mice and B1 cells (sorted as CD5⁺ B220^{low} CD19⁺ CD11b^{low}) isolated from the peritoneal cavities of young and old female B6 mice. Table 1 summarizes the increase in gene expression in ABCs.

| **Table 1** | |
|---|---|
| **Gene name** | **Average fold increase** |
| **Secreted proteins** | |
| **immunoglobulin heavy chain** | **37.1** |
| **CXCL10** | **33.9** |
| **CXCL3** | **16.4** |
| **CXCL19** | **13.9** |
| **CXCL9** | **13.4** |
| **CXCL8** | **11.0** |
| **granzyme A** | **14.4** |
| **perforin** | **8.8** |
| | |
| **Cell surface proteins** | **75.1** |
| **vascular cell adhesion molecule 1** | **6.1** |
| **integrin alpha X** | **53.2** |
| **integrin, alpha E** | **5.5** |
| **integrin alpha 2** | **4.7** |
| **IL-2 receptor beta chain** | **9.3** |
| **Fc receptor, IgE, high affinity I, gamma polypeptide** | **9.1** |
| | |
| **Proteins asociated with cytoskeleton and vesicle transport** | |
| **dynamin 3** | **28.4** |
| **kinesin family member C3** | **8.2** |
| **kinesin family member 5C** | **4.4** |
| **myosin IF** | **6.9** |
| **tropomyosin 2, beta** | **6.0** |
| **actin filament associated protein 1** | **4.4** |
| **formin 1** | **20.8** |
| **chimerin 2** | **20.2** |
| **syntaxin 3** | **22.1** |
| **phosphatase and actin regulator 2** | **12.2** |
| **dystrophin related protein 2** | **12.8** |
| **sprouty homolog 2 (Drosophila)** | **7.0** |
| **RAB39B** | **6.9** |
| **synaptotagmin-like 3** | **6.7** |
| **synaptopodin 2** | **5.7** |
| **coatomer protein complex, subunit zeta 2** | **4.2** |

Figure 5A shows a heat map of some of the differentially regulated transcripts, along with that of several control genes which were used to confirm the flow cytometric data. CD11c was among the transcripts that were substantially better expressed in the ABC population, in keeping with flow cytometric analysis. Several other strongly upregulated transcripts, such as those for immunoglobulin heavy chain (IgH) and Syndecan-1 (CD138), are characteristic of antibody secreting plasma cells. Although Syndecan-1 expression on ABCs was confirmed by flow cytometric analysis, it was lower than on fully differentiated plasma cells (data not shown). Also, in contrast to ABCs, plasma cells do not express most of the B cell characteristic markers such as B220, MHCII, CD80, CD86. Therefore ABCs are more likely to represent a unique population of plasmablasts, the precursors of plasma cells, than plasma cells themselves. CD11c expressing plasmablasts have been previously described in mice infected with intracellular bacteria (Racine et al., 2008). The phenotype of these cells is similar to that of ABCs, but not identical. For example, the plasmablasts found in mice infected with bacteria are CD5⁻CD11b^{high}, whereas ABCs are CD5⁺ and CD11b^{int}. The ABCs expressed other genes of interest and some unexpected genes. For example, ABCs contain mRNAs for several proinflammatory chemokines, such as CCL3, CXCL10, CCL19 and CXCL9 (Figure 5A), suggesting an activated status for this B cell population, an idea that is in keeping with their light scattering properties (Figure 1D). They also, unexpectedly contain mRNA for granzyme A and perforin (Figure 5A). This might suggest that the sorted ABC population used for these analyses was contaminated with cytotoxic T cells or NK cells. However, the list of genes increased in expression in ABCs did not include proteins characteristic of NK cells, NK T cells or CD8+ T cells (data not shown), and the criteria used to sort the ABCs would certainly have excluded NK cells and T cells (see Methods). In contrast, ABCs do not express cytokine genes at any higher level than the other B cell populations do.

Overall, the results from these analyses demonstrated that the gene expression by ABCs was clearly different from that of the other B cell population (Figure 5A). Importantly, although ABCs share many surface characteristics with peritoneal B1 cells, the gene array data strongly suggest these two cell types are quite distinct (Figure 5B). (Genealogical tree shown in Figure 5B was created by GeneSpring software based on gene expression profile of analyzed B cell populations.) Furthermore, the expression of almost 500 individual genes was greater by more than 2 fold between ABCs and all other B cell populations analyzed again indicating this was a unique B cell population (Table 2).

**Table 2.**

| | **UP** | **DOWN** |
|---|---|---|
| **ABCs vs. FO B spleen** | 1517 | 445 |
| **ABCs vs. old B1a, PEC** | 1150 | 737 |
| **ABCs vs. young B1a, PEC** | 1248 | 1026 |
| **ABCs vs. all populations together** | 491 | 34 |

Taken together, the flow cytometric and gene profile analysis show that ABCs represent a unique subpopulation of B cells which differ from B1 and FO B cells. Their expression of genes encoding a number of chemokines, cytotoxic proteins and costimulatory molecules suggests that these cells are activated and might be involved in the regulation of the immune response.

### Example 5.

This examples illustrates that TLR7 and MyD88 signaling are required for the development of ABCs.

To explore which factors might be required for female-specific development of ABCs, young and old female and male mice, deficient for specific genes, were examined for the presence of ABCs in the spleen. First, genes that have been implicated in the development of female-biased autoimmunity were tested. Considerable evidence suggests that lupus in human and lupus-like disease in mice is accompanied by higher-than-normal levels of IFNαβ in the affected individuals (Alarcon-Segovia et al., 1974; Hooks et al., 1979; Jorgensen et al., 2007). Evidence also suggests that the onset of autoimmunity is often associated with disregulation of Toll-like receptors (TLRs), key players of innate immurity involved in the recognition of pathogen-associated molecular structures (Berland et al., 2006; Deane et al., 2007). For instance, in Yaa mice, duplication of the TLR7 gene exacerbates lupus-like disease, although these data seem to be controversial (Deane et al., 2007; Santiago-Raber et al., 2008). Since the gene encoding TLR7 is located on a non-Lyonized part of the X chromosome, immune cells in female mice express higher levels of this receptor and have been shown to produce higher amount of IFNαβ in response to TLR7 agonist (Berghofer et al., 2006).

To test whether IFNαβ or TLR7 signaling is required for ABC accumulation in aged wild type female mice, young and old female mice deficient in the receptor for IFNαβ, IFNαR, or deficient in TLR7 were screened for the presence of ABCs in their spleens (Figure 6). (MyD88^{-/-} mice were generated by S. Akira and bred on in the National Jewish Health animal facility. IFN_{α/β}R-deficient mice were generated by M. Aguet and bred on in National Jewish animal facility.) TLR7^{-/-} aged female mice failed to accumulate ABCs while the number of ABCs in IFNαR^{-/-} mice was similar to that in wild type C57BL/6 mice. Likewise, ABCs did not accumulate in MyD88^{-/-} mice, which lack the key adaptor to initiate TLR7 signaling (Figure 6) while were found at normal frequency in TLR3-deficient mice (data not shown).

These findings suggested that the accumulation of ABCs requires signaling through TLR7 and MyD88. The fact that in IFNαR^{-/-} mice the number of ABCs was comparable to that in wild type mice was quite surprising considering that one of the main downstream effects of TLR7 signaling is production of IFNαβ. This indicates that, for ABC accumulation, TLR7 is acting via a pathway that does not require IFNαβ.

Since ABCs could not be found in aged TLR7 deficient mice it was hypothesized that chronic signaling through this receptor, but not through other TLRs, is sufficient to induce the accumulation of ABCs. To test this hypothesis, 12 week old C57BL/6 female mice were injected 3 times a week with low doses of TLR3, TLR4 or TLR7 agonists for two months. Two different TLR7 agonists were used for chronic intra peritoneal immunization of C57BL/6 male and female mice: 5 µg of 3M-012 or 50 µg of S-27609 (3M Pharmaceuticals, a gift from R. Kedl). Other TLR agonists were used at the following concentrations: 1 µg LPS (Escherichia coli 026; B6), 5 µg poly(I:C) (InvivoGen). For chronic immunization mice were immunized i.p. 3 times a week for 2-3 months. Following this, spleens were examined by flow cytometry for the presence of ABCs. As shown in Figure 7A TLR7, but not TLR3 or TLR4, stimulation led to the accumulation of ABCs, confirming the unique role of TLR7 in this process.

TLR7 is the only Toll-like receptor whose gene is encoded on the X-chromosome in mice (TLR8 is not expressed in mice), and injection of TLR7 agonist results in higher IFN production in females than in males (Berghofer et al., 2006), leading to the suggestion that TLR7 signaling in females is augmented in comparison to males. To test this idea, the present inventors compared the accumulation of ABCs, in response to chronic TLR7 stimulation, in the spleens of young C57BL/6 male and female mice.

In agreement with a previous study (Berghofer et al., 2006), the accumulation of ABCs in response to TLR7 stimulation was significantly higher in female than in male mice (Figure 7B), suggesting unequal expression of this receptor on cells in female and male mice.

Together, these data demonstrate that increased responsiveness to TLR7 stimulation in female mice results in accumulation of a unique CD11c⁺ B cell population and may account for the phenomenon of female-biased predisposition to autoimmune diseases.

### Example 6:

This Example shows that elderly human females contain an expanded population of ABCs.

Peripheral blood leukocytes were prepared from healthy human volunteers over the age of 60 (elderly) or under the age of 30 (young) and stained to identify the CD4⁻CD8⁻CD19⁺CD11c⁺ B cells. As shown in Figure 9, elderly females had increased numbers of ABCs in their blood compared with elderly males or young humans of either sex. The elevated numbers of ABCs in females as they age may contribute to the fact that females are more prone to autoimmune diseases such as lupus and rheumatoid arthritis than males.

The foregoing description of the present invention has been presented for purposes of illustration and description. The scope of the invention is defined by the appended claims.

### REFERENCES

Alarcon-Segovia, D., Ruiz-Gomez, J., Fishbein, E., and Bustamante, M.E. (1974). Interferon production by lymphocytes from patients with systemic lupus erythematosus. Arthritis Rheum 17, 590-592.
Beeson, P.B. (1994). Age and sex associations of 40 autoimmune diseases. The American journal of medicine 96, 457-462.
Berghofer, B., Frommer, T., Haley, G., Fink, L., Bein, G., and Hackstein, H. (2006). TLR7 ligands induce higher IFN-alpha production in females. J Immunol 177, 2088-2096. Berland, R., Fernandez, L., Kari, E., Han, J.H., Lomakin, I., Akira, S., Wortis, H.H., Kearney, J.F., Ucci, A.A., and Imanishi-Kari, T. (2006). Toll-like receptor 7-dependent loss of B cell tolerance in pathogenic autoantibody knockin mice. Immunity 25, 429-440. Bjornvold, M., Amundsen, S.S., Stene, L.C., Joner, G., Dahl-Jorgensen, K., Njolstad, P.R., Ek, J., Ascher, H., Gudjonsdottir, A.H., Lie, B.A., et al. (2006). FOXP3 polymorphisms in type 1 diabetes and coeliac disease. Journal of autoimmunity 27, 140-144.
Bonardelle, D., Benihoud, K., Kiger, N., and Bobe, P. (2005). B lymphocytes mediate Fas-dependent cytotoxicity in MRL/lpr mice. Journal of leukocyte biology 78, 1052-1059.
Bouillet, P., Metcalf, D., Huang, D.C., Tarlinton, D.M., Kay, T.W., Kontgen, F., Adams, J.M., and Strasser, A. (1999). Proapoptotic Bcl-2 relative Bim required for certain apoptotic responses, leukocyte homeostasis, and to preclude autoimmunity. Science (New York, N.Y 286, 1735-1738.
Carrel, L., and Willard, H.F. (2005). X-inactivation profile reveals extensive variability in X-linked gene expression in females. Nature 434, 400-404.
Chen, M., Huang, L., and Wang, J. (2007). Deficiency of Bim in dendritic cells contributes to overactivation of lymphocytes and autoimmunity. Blood 109, 4360-4367.
Cohen, P.L., Caricchio, R., Abraham, V., Camenisch, T.D., Jennette, J.C., Roubey, R.A., Earp, H.S., Matsushima, G., and Reap, E.A. (2002). Delayed apoptotic cell clearance and lupus-like autoimmunity in mice lacking the c-mer membrane tyrosine kinase. The Journal of experimental medicine 196, 135-140.
Cooper, G.S., F.W. Miller, and J.P. Pandey. 1999. The role of genetic factors in autoimmune disease: implications for environmental research. Environ Health Perspect 107 Suppl 5:693-700.
Culton, D.A., O'Conner, B.P., Conway, K.L., Diz, R., Rutan, J., Vilen, B.J., and Clarke, S.H. (2006). Early preplasma cells define a tolerance checkpoint for autoreactive B cells. J Immunol 176, 790-802.
Cutolo, M., Sulli, A., Capellino, S., Villaggio, B., Montagna, P., Seriolo, B., and Straub, R.H. (2004). Sex hormones influence on the immune system: basic and clinical aspects in autoimmunity. Lupus 13, 635-638.
Deane, J.A., Pisitkun, P., Barrett, R.S., Feigenbaum, L., Town, T., Ward, J.M., Flavell, R.A., and Bolland, S. (2007). Control of Toll-like Receptor 7 Expression Is Essential to Restrict Autoimmunity and Dendritic Cell Proliferation. Immunity 27, 801-810.
Diebold, S.S. (2008). Recognition of viral single-stranded RNA by Toll-like receptors. Advanced drug delivery reviews 60, 813-823.
Diebold, S.S., Massacrier, C., Akira, S., Paturel, C., Morel, Y., and Reis e Sousa, C. (2006). Nucleic acid agonists for Toll-like receptor 7 are defined by the presence of uridine ribonucleotides. European journal of immunology 36, 3256-3267.
Eaton-Bassiri, A.S., Mandik-Nayak, L., Seo, S.J., Madaio, M.P., Cancro, M.P., and Erikson, J. (2000). Alterations in splenic architecture and the localization of anti-doublestranded DNA B cells in aged mice. International immunology 12, 915-926.
Ehrhardt, G.R., Hijikata, A., Kitamura, H., Ohara, O., Wang, J.Y., and Cooper, M.D. (2008). Discriminating gene expression profiles of memory B cell subpopulations. J Exp Med.
Fairhurst, A.M., Hwang, S.H., Wang, A., Tian, X.H., Boudreaux, C., Zhou, X.J., Casco, J., Li, Q.Z., Connolly, J.E., and Wakeland, E.K. (2008). Yaa autoimmune phenotypes are conferred by overexpression of TLR7. European journal of immunology 38, 1971-1978. Fisher, G.H., Rosenberg, F.J., Straus, S.E., Dale, J.K., Middleton, L.A., Lin, A.Y., Strober, W., Lenardo, M.J., and Puck, J.M. (1995). Dominant interfering Fas gene mutations impair apoptosis in a human autoimmune lymphoproliferative syndrome. Cell 81, 935-946.
Gubbels Bupp, M.R., Jorgensen, T.N., and Kotzin, B.L. (2008). Identification of candidate genes that influence sex hormone-dependent disease phenotypes in mouse lupus. Genes and immunity 9, 47-56.
Guth, A., Detanico, T., Smith, D., Tung, K., Bonorino, C., and Wysocki, L. (2009). Spontaneous autoimmunity in mice that carry an IghV partial transgene: a required arginine in VHCDR3. Lupus 18, 299-308.
Hande, S., Notidis, E., and Manser, T. (1998). Bcl-2 obstructs negative selection of autoreactive, hypermutated antibody V regions during memory B cell development. Immunity 8, 189-198.
Haskins, K., Kubo, R., White, J., Pigeon, M., Kappler, J., and Marrack, P. (1983). The major histocompatibility complex-restricted antigen receptor on T cells. I. Isolation with a monoclonal antibody. The Journal of experimental medicine 157, 1149-1169.
Hill, N.J., Stotland, A., Solomon, M., Secrest, P., Getzoff, E., and Sarvetnick, N. (2007). Resistance of the target islet tissue to autoimmune destruction contributes to genetic susceptibility in Type 1 diabetes. Biology direct 2, 5.
Hoedemaekers, A., Bessereau, J.L., Graus, Y., Guyon, T., Changeux, J.P., Berrih-Aknin, S., van Breda Vriesman, P., and De Baets, M.H. (1998). Role of the target organ in determining susceptibility to experimental autoimmune myasthenia gravis. Journal of neuroimmunology 89, 131-141.
Hooks, J.J., Moutsopoulos, H.M., Geis, S.A., Stahl, N.I., Decker, J.L., and Notkins, A.L. (1979). Immune interferon in the circulation of patients with autoimmune disease. The New England journal of medicine 301, 5-8.
Hornung, V., Guenthner-Biller, M., Bourquin, C., Ablasser, A., Schlee, M., Uematsu, S., Noronha, A., Manoharan, M., Akira, S., de Fougerolles, A., et al. (2005). Sequence-specific potent induction of IFN-alpha by short interfering RNA in plasmacytoid dendritic cells through TLR7. Nature medicine 11, 263-270.
Izawa, T., Ishimaru, N., Moriyama, K., Kohashi, M., Arakaki, R., and Hayashi, Y. (2007). Crosstalk between RANKL and Fas signaling in dendritic cells controls immune tolerance. Blood 110, 242-250.
Jarvinen, P., and K. Aho. 1994. Twin studies in rheumatic diseases. Semin Arthritis Rheum 24:19-28.Johnson, S.A., Rozzo, S.J., and Cambier, J.C. (2002). Aging-dependent exclusion of antigen-inexperienced cells from the peripheral B cell repertoire. J Immunol 168, 5014-5023.
Jorgensen, T.N., Roper, E., Thurman, J.M., Marrack, P., and Kotzin, B.L. (2007). Type I interferon signaling is involved in the spontaneous development of lupus-like disease in B6.Nba2 and (B6.Nba2 x NZW)F(1) mice. Genes and immunity 8, 653-662.
Jung, S., Unutmaz, D., Wong, P., Sano, G., De los Santos, K., Sparwasser, T., Wu, S., Vuthoori, S., Ko, K., Zavala, F., et al. (2002). In vivo depletion of CDllc(+) dendritic cells abrogates priming of CD8(+) T cells by exogenous cell-associated antigens. Immunity 17, 211-220.
Kennedy, L.G., Will, R., and Calin, A. (1993). Sex ratio in the spondyloarthropathies and its relationship to phenotypic expression, mode of inheritance and age at onset. The Journal of rheumatology 20, 1900-1904.
Kono, D.H., and Theofilopoulos, A.N. (2000). Genetics of systemic autoimmunity in mouse models of lupus. International reviews of immunology 19, 367-387.
Kost, C.B., Holden, J.T., and Mann, K.P. (2008). Marginal zone B-cell lymphoma: A retrospective immunophenotypic analysis. Cytometry B Clin Cytom.
Larange, A., Antonios, D., Pallardy, M., and Kerdine-Romer, S. (2009). TLR7 and TLR8 agonists trigger different signaling pathways for human dendritic cell maturation. Journal of leukocyte biology 85, 673-683.
Lundy, S.K. (2009). Killer B lymphocytes: the evidence and the potential. Inflamm Res. Lyon, M.F. (1961). Gene action in the X-chromosome of the mouse (Mus musculus L.). Nature 190, 372-373.
McDevitt, H.O., and Bodmer, W.F. (1974). HL-A, immune-response genes, and disease. Lancet 1, 1269-1275.
Miller, R.L., Gerster, J.F., Owens, M.L., Slade, H.B., and Tomai, M.A. (1999). Imiquimod applied topically: a novel immune response modifier and new class of drug. International journal of immunopharmacology 21, 1-14.
Morice, W.G., Kurtin, P.J., Hodnefield, J.M., Shanafelt, T.D., Hoyer, J.D., Remstein, E.D., and Hanson, C.A. (2008). Predictive value of blood and bone marrow flow cytometry in B-cell lymphoma classification: comparative analysis of flow cytometry and tissue biopsy in 252 patients. Mayo Clin Proc 83, 776-785.
Nicholas, M.W., Dooley, M.A., Hogan, S.L., Anolik, J., Looney, J., Sanz, I., and Clarke, S.H. (2008). A novel subset of memory B cells is enriched in autoreactivity and correlates with adverse outcomes in SLE. Clinical immunology (Orlando, Fla 126, 189-201.
Parsa, A., Lovett, D.H., Peden, E.A., Zhu, L., Seldin, M.F., and Criswell, L.A. (2005). Renin-angiotensin system gene polymorphisms predict the progression to renal insufficiency among Asians with lupus nephritis. Genes and immunity 6, 217-224. Pisitkun, P., Deane, J.A., Difilippantonio, M.J., Tarasenko, T., Satterthwaite, A.B., and Bolland, S. (2006). Autoreactive B cell responses to RNA-related antigens due to TLR7 gene duplication. Science (New York, N.Y 312, 1669-1672.
Racine, R., Chatterjee, M., and Winslow, G.M. (2008). CD11c expression identifies a population of extrafollicular antigen-specific splenic plasmablasts responsible for CD4 T-independent antibody responses during intracellular bacterial infection. J Immunol 181, 1375-1385.
Radic, M.Z., K. Shah, W. Zhang, Q. Lu, G. Lemke, and G.M. Hilliard. 2006. Heterogeneous nuclear ribonucleoprotein P2 is an autoantibody target in mice deficient for Mer, Axl, and Tyro3 receptor tyrosine kinases. J Immunol 176:68-74.
Rascu, A., Repp, R., Westerdaal, N.A., Kalden, J.R., and van de Winkel, J.G. (1997). Clinical relevance of Fc gamma receptor polymorphisms. Annals of the New York Academy of Sciences 815, 282-295.
Roubinian, J.R., Papoian, R., and Talal, N. (1977). Androgenic hormones modulate autoantibody responses and improve survival in murine lupus. The Journal of clinical investigation 59, 1066-1070.
Rubtsov, A.V., Swanson, C.L., Troy, S., Strauch, P., Pelanda, R., and Torres, R.M. (2008). TLR agonists promote marginal zone B cell activation and facilitate T-dependent IgM responses. J Immunol 180, 3882-3888.
Santiago-Raber, M.L., Kikuchi, S., Borel, P., Uematsu, S., Akira, S., Kotzin, B.L., and Izui, S. (2008). Evidence for genes in addition to Tlr7 in the Yaa translocation linked with acceleration of systemic lupus erythematosus. J Immunol 181, 1556-1562.
Scott, R.S., McMahon, E.J., Pop, S.M., Reap, E.A., Caricchio, R., Cohen, P.L., Earp, H.S., and Matsushima, G.K. (2001). Phagocytosis and clearance of apoptotic cells is mediated by MER. Nature 411, 207-211.
Smith-Bouvier, D.L., Divekar, A.A., Sasidhar, M., Du, S., Tiwari-Woodruff, S.K., King, J.K., Arnold, A.P., Singh, R.R., and Voskuhl, R.R. (2008). A role for sex chromosome complement in the female bias in autoimmune disease. The Journal of experimental medicine 205, 1099-1108.
Talal, N. 1978. Natural history of murine lupus. Modulation by sex hormones. Arthritis Rheum 21:S58-63.
Tsuchiya, N., and Kyogoku, C. (2005). Role of Fc gamma receptor IIb polymorphism in the genetic background of systemic lupus erythematosus: insights from Asia. Autoimmunity 38, 347-352.
Wandstrat, A.E., Nguyen, C., Limaye, N., Chan, A.Y., Subramanian, S., Tian, X.H., Yim, Y.S., Pertsemlidis, A., Garner, H.R., Jr., Morel, L., and Wakeland, E.K. (2004). Association of extensive polymorphisms in the SLAM/CD2 gene cluster with murine lupus. Immunity 21, 769-780.
Wang, H., Nicholas, M.W., Conway, K.L., Sen, P., Diz, R., Tisch, R.M., and Clarke, S.H. (2006). EBV latent membrane protein 2A induces autoreactive B cell activation and TLR hypersensitivity. J Immunol 177, 2793-2802.
Whitacre, C.C. (2001). Sex differences in autoimmune disease. Nature immunology 2, 777-780.
Wille-Reece, U., Wu, C.Y., Flynn, B.J., Kedl, R.M., and Seder, R.A. (2005). Immunization with HIV-1 Gag protein conjugated to a TLR7/8 agonist results in the generation of HIV-1 Gag-specific Th1 and CD8+ T cell responses. J Immunol 174, 7676-7683.
Yen, Z.C., Meyer, I.M., Karalic, S., and Brown, C.J. (2007). A cross-species comparison of X-chromosome inactivation in Eutheria. Genomics 90, 453-463.
Zandman-Goddard, G., E. Peeva, and Y. Shoenfeld. 2007. Gender and autoimmunity. Autoimmun Rev 6:366-372.

## Claims

1. A composition for use in treating an autoimmune disease in a subject by reducing the activity of autoimmune-associated B cells (ABCs) present in the subject, wherein the ABCs are **characterized by** at least the presence of marker CD11c, wherein the composition comprises an agent that is an antibody or antibody fragment that specifically binds to a protein expressed by the ABCs and wherein the protein is CD11c, and
at least one antibody or antibody fragment that specifically binds an additional protein selected from the group consisting of CD11b, B220, CD19, a surface Ig, CD80, CD86, MHC class II, CD5, CCL3, CXCL10, CCL19, CXCL9, granzyme A, and perforin.

2. A method of diagnosing an autoimmune disease in a subject, comprising:
detecting the presence of autoimmune-associated B cells ("ABCs") in a test sample, wherein the ABCs comprise B cells that express the protein CD11c and one or more additional marker proteins, wherein detecting the presence of ABCs in the sample comprises detecting the cells that express CD11c and one or more additional marker proteins by detecting the expression of CD11c and the one or more additional marker proteins in the test sample, wherein expression of CD11c and the one or more additional marker proteins indicates the presence of ABCs in the test sample; and
wherein the presence of ABCs in the sample at an elevated level as compared to a baseline level established from a control sample, identifies the subject as having or likely to develop the autoimmune disease.

3. The method of claim 2, wherein the one or more additional marker proteins are selected from the group consisting of: CD11b, B220, CD19 and a cell surface Immunoglobulin Ig, wherein the surface Ig is selected from the group consisting of: IgG, IgM, IgA and IgE.

4. The method of claim 2, wherein the one or more additional marker proteins are selected from the group consisting of: CD80, CD86, MHC class II, CD5, CCL3, CXCL10, CCL19, CXCL9, granzyme A and perforin.

5. The method of claim 2, wherein the ABCs express low levels of CD21 as compared to other B cells.

6. The method of claim 2, wherein detecting the expression of CD11c and the one or more additional marker proteins comprises co-immunostaining the cells with an antibody or antibody fragment that specifically recognizes CD11c, and an antibody or antibody fragment that specifically recognizes the additional marker protein.

7. The method of claim 2, wherein detecting the expression of CD11c and the one or more additional marker proteins comprises detecting the mRNA levels of CD11c and the additional marker protein.

8. The method of claim 2, further comprising determining the frequency of the cells that express the protein CD11c and the additional marker protein.

9. The method of claim 2, wherein the test sample is a blood sample comprising peripheral blood cells.

10. Use of a composition in an in vitro method for the diagnosis of an autoimmune disease, the composition comprising an agent that is an antibody or antibody fragment that specifically binds to a protein expressed by autoimmune-associated B cells (ABCs) wherein the protein is CD11c and wherein the composition comprises at least one antibody or antibody fragment that specifically binds an additional protein selected from the group consisting of CD11b, B220, CD19, a surface Ig, CD80, CD86, MHC class II, CD5, CCL3, CXCL10, CCL19, CXCL9, granzyme A, and perforin.

11. A method to evaluate the efficacy of a treatment of an autoimmune disease in a subject, comprising
a) detecting the presence of autoimmune-associated B cells (ABCs) **characterized by** at least the presence of marker CD11c in a test sample taken from the subject before administering the treatment,
b) detecting the presence of ABCs in a test sample taken from the subject after administering the treatment; wherein detecting the presence of ABCs in the sample before and after administering treatment comprises detecting B cells that express CD11c and one or more additional marker proteins by detecting the expression of CD11c and the one or more additional markerproteins in the test sample, wherein expression of CD11c and the one or more additional marker proteins indicates the presence of ABCs in the test sample; and
c) comparing the level of ABCs in the test sample taken from the subject before administering the treatment to the level of ABCs in the test sample taken from the subject after administering the treatment; wherein ABCs comprise B cells that express CD11c.

12. The method of any one of the previous claims 2-9 and 11, wherein the autoimmune disease is selected from the group consisting of: lupus, rheumatoid arthritis, multiple sclerosis, insulin dependent diabetes mellitis, myasthenia gravis, Grave's disease, autoimmune hemolytic anemia, autoimmune thrombocytopenia purpura, Goodpasture's syndrome, pemphigus vulgaris, acute rheumatic fever, post-streptococcal glomerulonephritis, and polyarteritis nodosa.

## Patentansprüche

1. Zusammensetzung zur Verwendung in der Behandlung einer Autoimmunerkrankung in einem Individuum durch Reduzieren der Aktivität von autoimmun-zugeordneten B-Zellen (ABCs), die in dem Individuum vorliegen, wobei die ABCs durch zumindest der Anwesenheit des Markers CD11c gekennzeichnet sind, wobei die Zusammensetzung ein Mittel umfasst, das ein Antikörper oder Antikörperfragment ist, das sich speziell an ein Protein bindet, das durch die ABCs exprimiert wird, und wobei das Protein CD11c ist, und zumindest ein Antikörper oder Antikörperfragment, der/das sich an ein zusätzliches Protein bindet, das aus der Gruppe bestehend aus CD11b, B220, CD19, einem Oberflächen-Ig, CD80, CD86, MHC-Klasse II, CD5, CCL3, CXCL10, CCL19, CXCL9, Granzym A und Perforin ausgewählt wird.

2. Verfahren zur Diagnose einer Autoimmunerkrankung in einem Individuum, umfassend:
Nachweis der Anwesenheit von autoimmun-zugeordneten B-Zellen ("ABCs") in einer Probe, wobei die ABCs B-Zellen, die das Protein CD11c exprimieren, und ein oder mehrere zusätzliche Markerproteine umfassen, wobei der Nachweis der Anwesenheit von ABCs in der Probe den Nachweis der CD11c exprimierenden Zellen und ein oder mehrere zusätzliche Markerproteine durch Nachweis der Expression von CD11c und dem einen oder den mehreren zusätzlichen Markerproteinen in der Probe umfasst, wobei die Expression von CD11c und dem einen oder den mehreren zusätzlichen Markerproteinen die Anwesenheit von ABCs in der Probe anzeigt; und
wobei die Anwesenheit von ABCs in der Probe bei einem erhöhten Spiegel, der mit einem von einer Kontrollprobe festgelegten Grundspiegel verglichen wird, identifiziert, ob das Individuum die Autoimmunerkrankung aufweist oder sie wahrscheinlich entwickeln wird.

3. Verfahren nach Anspruch 2, wobei das eine oder die mehreren zusätzlichen Markerproteine ausgewählt sind aus der Gruppe bestehend aus: CD11b, B220, CD19 und einer Zelloberfläche-Immunoglobulin Ig, wobei die Oberfläche Ig ausgewählt ist aus der Gruppe bestehend aus: IgG, IgM, IgA und IgE.

4. Verfahren nach Anspruch 2, wobei das eine oder die mehreren zusätzlichen Markerproteine ausgewählt sind aus der Gruppe bestehend aus: CD80, CD86, MHC-Klasse II, CD5, CCL3, CXCL10, CCL19, CXCL9, Granzym A und Perforin.

5. Verfahren nach Anspruch 2, wobei die ABCs niedrige Spiegel von CD21 im Vergleich zu anderen B-Zellen exprimieren.

6. Verfahren nach Anspruch 2, wobei der Nachweis der Expression von CD11c und dem einen oder den mehreren zusätzlichen Markerproteinen das Co-Immunanfärben der Zellen mit einem Antikörper oder Antikörperfragment umfasst, der/das speziell CD11c erkennt, und einem Antikörper oder Antikörperfragment, der/das speziell die zusätzlichen Markerproteine erkennt.

7. Verfahren nach Anspruch 2, wobei der Nachweis der Expression von CD11c und dem einen oder den mehreren zusätzlichen Markerproteinen den Nachweis der mRNA-Spiegel von CD11c und den zusätzlichen Markerproteinen umfasst.

8. Verfahren nach Anspruch 2, ferner umfassend das Bestimmen der Frequenz der Zellen, die das Protein CD11c und das zusätzliche Markerprotein exprimieren.

9. Verfahren nach Anspruch 2, wobei die Probe eine Blutprobe ist, die periphere Blutzellen umfasst.

10. Verwendung einer Zusammensetzung in einem In-vitro-Verfahren zur Diagnose einer Autoimmunerkrankung, wobei die Zusammensetzung ein Mittel umfasst, das ein Antikörper oder Antikörperfragment ist, der/das sich speziell an ein Protein bindet, das durch die autoimmun-zugeordneten B-Zellen (ABCs) exprimiert wird, wobei das Protein CD11c ist, und wobei die Zusammensetzung zumindest einen Antikörper oder Antikörperfragment umfasst, der/das sich an ein zusätzliches Protein bindet, das aus der Gruppe bestehend aus CD11b, B220, CD19, einem Oberflächen-Ig, CD80, CD86, MHC-Klasse II, CD5, CCL3, CXCL10, CCL19, CXCL9, Granzym A und Perforin ausgewählt wird.

11. Verfahren zur Bewertung der Wirksamkeit einer Behandlung einer Autoimmunerkrankung in einem Individuum, umfassend
a) Nachweis der Anwesenheit von autoimmun-zugeordneten B-Zellen (ABCs), **gekennzeichnet durch** zumindest der Anwesenheit des Markers CD11c in einer Probe, die vor Verabreichen der Behandlung von dem Individuum entnommen wird.
b) Nachweis der Anwesenheit von ABCs in einer Probe, die nach Verabreichen der Behandlung von dem Individuum entnommen wird; wobei der Nachweis der Anwesenheit von ABCs in der Probe vor und nach Verabreichen der Behandlung den Nachweis von B-Zellen, die CD11c und einen oder mehrere zusätzliche Markerproteine exprimieren, durch den Nachweis der Expression von CD11c und dem einem oder den mehreren zusätzlichen Markerproteinen in der Probe umfasst, wobei die Expression von CD11c und dem einen oder den mehreren zusätzlichen Markerproteinen die Anwesenheit von ABCs in der Probe anzeigt; und
c) Vergleichen des Spiegels von ABCs in der Probe, die dem Individuum vor dem Verabreichen der Behandlung entnommen wird, mit dem Spiegel von ABCs in der Probe, die dem Individuum nach dem Verabreichen der Behandlung entnommen wird; wobei die ABCs B-Zellen, die CD11c exprimieren, umfasst.

12. Verfahren nach einem der vorhergehenden Ansprüche 2 - 9 und 11, wobei die Autoimmunerkrankung ausgewählt ist aus der Gruppe bestehend aus Lupus, rheumatoider Arthritis, Multipler Sclerose, insulinabhängiger Diabetes mellitus, Myasthenia gravis, Morbus Basedow, autoimmuner hämolytischer Anämie, autoimmuner Thrombocytopenia purpura, Goodpasture-Syndrom, Pemphigus vulgaris, akutem rheumatischem Fieber, Poststreptokokken-Glomerulonephritis und Polyarteritis nodosa.

## Revendications

1. Composition pour utilisation dans le traitement d'une maladie auto-immune chez un sujet par réduction de l'activité des cellules B associées à l'auto-immunité (ABC) présentes dans le sujet, les ABC étant **caractérisées par** au moins la présence du marqueur CD11c, la composition comprenant un agent qui est un anticorps ou un fragment d'anticorps qui se lie spécifiquement à une protéine exprimée par les ABC et la protéine étant CD11c, et au moins un anticorps ou fragment d'anticorps qui se lie spécifiquement à une protéine supplémentaire sélectionnée dans le groupe constitué de CD11b, B220, CD19, une Ig de surface, CD80, CD86, MHC classe II, CD5, CCL3, CXCL10, CCL19, CXCL9, granzyme A et perforine.

2. Procédé de diagnostic d'une maladie auto-immune chez un sujet, comprenant :
la détection de la présence de cellules B associées à l'auto-immunité (ABC) dans un échantillon pour essai, les ABC comprenant des cellules B qui expriment la protéine CD11c et une ou plusieurs protéines marqueuses supplémentaires, où la détection de la présence d'ABC dans l'échantillon comprend la détection des cellules qui expriment CD11c et une ou plusieurs protéines marqueuses supplémentaires par détection de l'expression de CD11c et de la ou des protéines marqueuses supplémentaires dans l'échantillon pour essai, où l'expression de CD11c et de la ou des protéines marqueuses supplémentaires indique la présence d'ABC dans l'échantillon pour essai ; et
où la présence d'ABC dans l'échantillon à un niveau élevé par rapport à un niveau de base établi à partir d'un échantillon de référence identifie le sujet comme ayant ou étant susceptible de contracter la maladie auto-immune.

3. Procédé selon la revendication 2, dans lequel la ou les protéines marqueuses supplémentaires sont sélectionnées dans le groupe constitué de : CD11b, B220, CD19 et une immunoglobuline Ig de surface cellulaire, l'Ig de surface étant sélectionnée dans le groupe constitué de : IgG, IgM, IgA et IgE.

4. Procédé selon la revendication 2, dans lequel la ou les protéines marqueuses supplémentaires sont sélectionnées dans le groupe constitué de : CD80, CD86, MHC classe II, CD5, CCL3, CXCL10, CCL19, CXCL9, granzyme A et perforine.

5. Procédé selon la revendication 2, dans lequel les ABC expriment de bas niveaux de CD21 par rapport aux autres cellules B.

6. Procédé selon la revendication 2, dans lequel la détection de l'expression de CD11c et d'une ou plusieurs protéines marqueuses supplémentaires comprend la co-immunocoloration des cellules avec un anticorps ou fragment d'anticorps qui reconnaît spécifiquement CD11c, et un anticorps ou fragment d'anticorps qui reconnaît spécifiquement la protéine marqueuse supplémentaire.

7. Procédé selon la revendication 2, dans lequel la détection de l'expression de CD11c et de la ou des protéines marqueuses supplémentaires comprend la détection des niveaux mRNA de CD11c et de la protéine marqueuse supplémentaire.

8. Procédé selon la revendication 2, comprenant en outre la détermination de la fréquence des cellules qui expriment la protéine CD11c et la protéine marqueuse supplémentaire.

9. Procédé selon la revendication 2, dans lequel l'échantillon pour essai est un échantillon de sang comprenant des cellules du sang périphérique.

10. Utilisation d'une composition dans un procédé in vitro pour le diagnostic d'une maladie auto-immune, la composition comprenant un agent qui est un anticorps ou fragment d'anticorps qui se lie spécifiquement à une protéine exprimée par des cellules B associées à l'auto-immunité (ABC), la protéine étant CD11c et la composition comprenant au moins un anticorps ou fragment d'anticorps qui se lie spécifiquement à une protéine supplémentaire sélectionnée dans le groupe constitué de CD11b, B220, CD19, une Ig de surface, CD80, CD86, MHC classe II, CD5, CCL3, CXCL10, CCL19, CXCL9, granzyme A et perforine.

11. Procédé pour évaluer l'efficacité d'un traitement d'une maladie auto-immune chez un sujet, comprenant :
a) la détection de la présence de cellules B associées à l'auto-immunité (ABC) **caractérisées par** au moins la présence de marqueur CD11c dans un échantillon pour essai prélevé sur le sujet avant l'administration du traitement,
b) la détection de la présence d'ABC dans un échantillon pour essai prélevé sur le sujet après administration du traitement, la détection de la présence d'ABC dans l'échantillon avant et après administration du traitement comprenant la détection des cellules B qui expriment CD11c et une ou plusieurs protéines marqueuses supplémentaires par détection de l'expression de CD11c et de la ou des protéines marqueuses supplémentaires dans l'échantillon pour essai, où l'expression de CD11c et de la ou des protéines marqueuses supplémentaires indique la présence d'ABC dans l'échantillon pour essai ; et
c) la comparaison du niveau d'ABC dans l'échantillon pour essai prélevé sur le sujet avant administration du traitement au niveau d'ABC dans l'échantillon pour essai prélevé sur le sujet après administration du traitement, les ABC comprenant des cellules B qui expriment CD11c.

12. Procédé selon l'une quelconque des revendications précédentes 2-9 et 11, dans lequel la maladie auto-immune est sélectionnée dans le groupe constitué de : lupus, polyarthrite rhumatoïde, sclérose en plaques, diabète sucré insulinodépendant, myasthénie grave, maladie de Grave, anémie hémolytique auto-immune, purpura thrombopénique auto-immun, syndrome de Goodpasture, pemphigus vulgaire, fièvre rhumatismale aiguë, glomérulonéphrite post-streptococcique et polyartérite noueuse.
